(19) Europäisches Patentamt
European Patent Office
Office européen des brevets

(11) Publication number: 0 256 843
A1

(12) # EUROPEAN PATENT APPLICATION

(21) Application number: 87307114.6

(22) Date of filing: 11.08.87

(51) Int. Cl.³: C 12 N 15/00
C 12 P 21/02

(30) Priority: 11.08.86 US 895154
18.11.86 US 932037

(43) Date of publication of application:
24.02.88 Bulletin 88/8

(84) Designated Contracting States:
AT BE CH DE ES FR GB GR IT LI LU NL SE

(71) Applicant: CETUS CORPORATION
1400 Fifty-Third Street
Emeryville California 94608(US)

(72) Inventor: Devlin, James Joseph
522 Silverado Drive
Lafayette California 94549(US)

(72) Inventor: Devlin, Patricia Egan
522 Silverado Drive
Lafayette California 94549(US)

(72) Inventor: Kawasaki, Ernest Seigo
260 San Mateo Street
Richmond California 94804(US)

(72) Inventor: Warren, Mary Kim
486 Joaquin Avenue
San Leandro California 94577(US)

(74) Representative: Bizley, Richard Edward et al,
BOULT, WADE & TENNANT 27 Furnival Street
London EC4A 1PQ(GB)

(54) **Expression of g-csf and muteins thereof and their use.**

(57) Human recombinant G-CSF (hr-G-CSF), an mRNA fraction encoding hr-G-CSF, a DNA sequence encoding hr-G-CSF, modified DNA sequences encoding hr-G-CSF, that are expressed at high levels in recombinant hosts are disclosed. Modifications to the 5′ DNA sequence encoding mature mG-CSF are also disclosed. The modifications to the third position nucleotide of at least one in the 5′ region codon of the DNA sequence of G-CSF result in enhanced expression of mG-CSF protein in recombinant host cells, without changing the amino acid sequence of the protein. Other modifications yield muteins of G-CSF in which the $NH_2$-terminal amino acid is not methionine when produced from a recombinant host cell. Expression vectors carrying the altered mG-CSF DNA sequence are disclosed and claimed.

EP 0 256 843 A1

# EXPRESSION OF G-CSF AND MUTEINS THEREOF AND THEIR USE

The invention concerns the field of recombinant DNA. In particular, the invention concerns the isolation of nucleic acid sequences encoding granulocyte colony-stimulating factor (G-CSF) and the expression of G-CSF in various host cells. The invention concerns modified DNA sequences encoding G-CSF and muteins thereof that are efficiently expressed under the control of promoters operable in E. coli, expression vectors utilizing the modified DNA sequences for the efficient expression of G-CSF in E. coli and Gram-negative hosts known to exchange genetic material with E. coli, host organisms including microorganisms transformed with the expression vectors and G-CSF produced thereby.

Interest in the growth and differentiation of granulocytes and macrophages from bone marrow progenitors has prompted the study of the colony-stimulating factors (CSFs) that regulate these processes. The subject has been recently reviewed by D. Metcalf in Blood, 67:257 (1986). One of these factors, termed granulocyte-CSF (G-CSF) stimulates the in vitro growth of predominantly granulocyte colonies from bone marrow stem cells. The availability of cell lines that produce CSFs has been instrumental in the characterization of G-CSF; however, proper interpretation of this work requires the precise identification of the CSF released by these cells. One such cell line, the MIA PaCa-2, derived from a human pancreatic carcinoma, reportedly produces both the macrophage-specific CSF-1 and a CSF that can induce the formation of primarily granulocyte colonies in seven day bone marrow cell cultures. Wu, M. C., J. Clin. Invest., 67:1588 (1981). A CSF-1 cDNA clone has been recently isolated from this cell line, Kawasaki et al., Science, 230:291 (1985). They also showed, by translation of sucrose gradient fractionated mRNA in Xenopus laevis oocytes, that a transcript that sedimented slower than the 18s marker encoded a CSF that induced the proliferation of murine bone marrow cells; however, the CSF-1 clone hybridized most strongly to mRNA in the fractions that sedimented faster than the 18s marker.

Human G-CSF causes the differentiation of granulocytes and monocytes from bone marrow progenitor cells. It also induces some leukemia cells to differentiate into more mature cell types. Thus, human G-CSF may be useful in regeneration of important blood cell populations in immunologically compromised patients and patients with anemic disorders or infections. G-CSF may also be useful in inhibiting leukemia by causing leukemia cells to differentiate to a non-dividing state, thereby checking the uncontrolled proliferation of these cell types. Myeloid leukemia cells are known to have receptors for G-CSF, for example. Science, 323:7 (1986).

Souza et al. have isolated, cloned, and expressed a gene encoding G-CSF, which has no significant homology to GM-CSF, in an unidentified strain of *E. coli* using a modified temperature sensitive runaway plasmid with a λ phage PL promoter and synthetic DNA fragment containing an initiation codon, followed by the sequence encoding the mature form of unaltered G-CSF. No data on expression levels are, however, disclosed. Souza et al., Science, 232:61-65 (1986).

In addition, a colony stimulating factor having the same N-terminal 20 amino acids as that of Souza et al. was isolated from a cultured human oral carcinoma as disclosed in European Patent Publication 0169,566 to Nagata et al. This clone designated CHU-2, has an additional sequence encoding Val-Ser-Glu just prior to the $cys_{36}$ residue of the protein encoded by the DNA sequence cloned from MIA PaCa-2, as described herein below. No information is given in either of the above-mentioned disclosures as to the levels of expression in recombinant hosts, particulaly *E. coli*. Nagata et al. subsequently reported a cDNA sequence that lacks the sequence encoding the above-mentioned additional tri-peptide sequence. Nagata et al., EMBO Journal 5:3, 575-581 (1986).

Reportedly, both of these recombinantly produced G-CSFs have $NH_2$ terminal methionines as a result of expression in the recombinant bacterial host *E. coli*. Such $NH_2$-terminal methionines are generally known to be immunogenic when the protein is administered parenterally to a mammalian host.

A number of factors are involved in obtaining maximal expression of a protein in a recombinant host. Among these factors are optimization of the physiological conditions for growth of the host cell by changes to the culture media in which the host grows. Also important is the selection and use of strong promoter and ribosome binding sites (RBS) that are operable in the expression vector to be used. The elimination of attenuator regions if they occur in the region of regulated promoters such as the tryptophan operon is also desirable.

A more subtle factor in maximizing protein expression in a recombinant host relates to codon utilization. In brief, codon utilization may be described as the ability of a particular recombinant host organism to translate some codons encoding a particular amino acid more quickly and accurately than other different codons encoding the same amino acid. Thus, although the trinucleotide code specifying a particular amino acid is degenerate, i.e., more than one trinucleotide sequence can encode a particular amino acid, all codons encoding a particular amino acid are not utilized by the host with equal efficiency. Enhanced expression may be obtained by changing the nucleotide sequence of a gene in a recombinant host to the host preferred codons without changing the amino acid sequence of the protein encoded thereby. Additionally, changes in the amino acid sequence of the $NH_2$ terminus may effect processing of the $NH_2$-terminal methionine mentioned above.

Another subtlety in the enhancement of gene expression in an expression vector in a recombinant host relates to secondary structure formation in the mRNA sequence to be expressed. It has been shown, for example, that certain "stem and loop" secondary structures placed in proximity to the 3' terminus of a gene, lead to increased stability of the mRNA transcript and increased expression of the protein encoded thereby, Wong et al., Proc. Nat. Acad. Sci. (USA), 83:10, 32-33 (1986). However, it has been suggested that formation of stable secondary structures at the 5' end of an mRNA transcript may block efficient translation thereof, leading to reduced yield of the protein encoded by the gene. The influence of mRNA secondary structure on translation has been discussed in Iserentant & Fiers, Gene, 9:1-12 (1980) and Buell et al., Nucleic Acid Research, 13:1923-1938 (1985). DeLamarter, J. F., et al., EMBO Journal, 4,10:2575-2581 (1985) have reported that the yield of GM-CSF has been increased in *E. coli* by means of altering third position deoxyribocytosine to deoxyriboadenines in the first ten of the 5' codons of the DNA encoding mature GM-CSF, and attribute the increase in protein synthesis to the altered 5' end of the mRNA transcript.

It would be desirable to obtain large amounts of G-CSF for development of therapies utilizing the granulocyte stimulating and granulocyte-macrophage stimulating characteristics of this colony stimulating factor. Furthermore, it would be desirable to obtain forms of G-CSF that lack $NH_2$-terminal methionine when expressed in the recombinant host, and particularly in *E. coli*.

The present invention makes available G-CSF in large quantities by producing it at high levels in a number of hosts.

The invention discloses that a previously isolated mRNA fraction from MIA PaCa-2 cells having high bone marrow proliferative activity encodes G-CSF. The invention encompasses the G-CSF mRNA. In addition, the invention encompasses a cDNA sequence made from this mRNA fraction that encodes the G-CSF gene.

The invention also includes DNA sequences encoding mature G-CSF; these DNA sequences have been altered to facilitate expression of G-CSF in recombinant hosts. In addition, DNA sequence encoding mature G-CSF or a mutein thereof lacking $NH_2$-terminal methionine when expressed in a recombinant host, are disclosed.

At present, investigators have encountered great difficulty in obtaining significant expression of mature G-CSF in *E. coli* hosts under $P_L$ promoter gene N-RBS control or tryptophan (Trp) promoter control when the unaltered nucleotide sequence of the mature G-CSF is used. The inventors have determined that, when under $P_L$ gene N-RBS control in *E. coli*, steady state levels of mRNA encoding G-CSF are detectable by Northern analysis as described further herein. Under the control of the Trp promoter, no steady state G-CSF mRNA is found in *E. coli* by the same method. Surprisingly, in light of the work of Delemarter, et al. which suggests that alterations of the 5' end of the GM-CSF gene affects secondary structure of the GM-CSF mRNA sequence and the translation thereof, the inventors have found that alterations in the 5' region of the G-CSF gene leads not only to increased expression of G-CSF protein in both $P_L$ gene N-RBS and Trp controlled expression vectors in *E. coli*, it also leads to increased levels of mRNA steady state transcript in Trp controlled expression vectors in *E. coli*.

Figure 1A is a plot of bone marrow proliferation activity obtained for *Xenopus* oocyte supernatants made from the fractionated MIA PaCa-2 mRNA.

Figure 1B is a dot blot of MIA PaCa-2 mRNA fractions with a CSF-1-specific probe.

Figure 2 is a Northern blot of the RNA obtained from MIA PaCa-2 cells (lane 1), LD-1 cells (lane 2), and 5637 cells (lane 3), probed with two different 24-mer probes. In 2A the probe was 5'-ATGGCTGGACCTGCCACCCACAGC-3', which hybridized to RNA of all three lanes. In 2B the oligomer probe was 5'-AGAAGCTGGTGAGTGAGTGTGCCA-3'. The nine nucleotides underlined appear only in the CHU-2 sequence that purports to be G-CSF (Nagata et al., *supra*). This probe did not hybridize to any of the cell lines tested under stringent conditions.

Figure 3A is a plot of the bone marrow proliferation activity of the supernatants collected from *Xenopus laevis* oocytes 40 hours after injection of the pooled MIA PaCa-2 RNA fractions. Activity was measured by the murine bone marrow cell proliferation assay described in the examples below.

Figure 3B is a Northern blot of the pooled MIA PaCa-2 mRNA fractions using a G-CSF specific $\gamma^{32}P$ labeled oligodeoxyribonucleotide probe.

Figure 4 shows the complete sequence of G-CSF cDNA obtained from the sequencing of the *Bam*HI digest of plasmid pP12 and deduced amino acid sequence encoded thereby. The arrow indicates the position of the nine nucleotide sequence GTGATGGAG of the Nagata et al. clone.

Figure 5 is a schematic illustration showing the prepartion of plasmid pPD1 and the placement of a HindIII restriction site and ATG codon in the G-CSF sequence.

Figure 6 is a schematic illustration of the prepartion of plasmid pPD2 in which the 3' untranslated region of the G-CSF gene is excised and transcription termination and mRNA stability are placed under control of the positive retroregulatory element.

Figure 7 is a schematic illustration of the preparation of plasmid pJD1 in which G-CSF expression is under the control of the $P_L$ promoter and gene N-ribosome binding site.

Figure 8 is a schematic illustration of the preparation of pJD4, pJD4A and pJD4B.

Figure 9 is a schematic illustration of the preparation of pPD5.

Figure 10 is a Northern blot of mRNA from pJD1 and pPD2 probed with a G-CSF specific oligodeoxynucleotide probe.

Figure 11 shows the DNA sequence and N-terminal amino acid sequence encoded by native G-CSF, pJD4B, pPD5 and pJD4A. The ATG codon encoding Met was added to native G-CSF and does not occur in the cDNA sequence obtained from MIA PaCa-2 transcripts.

Figure 12 is an SDS gel showing expression of G-CSF in induced and uninduced *E. coli* host carrying pJD4A and pJD4B.

Figure 13 is an SDS gel showing expression of G-CSF in induced and uninduced *E. coli* host carrying pPD5.

Figure 14 is a schematic illustration of the preparation of plasmid pPD6 in which $\nabla_1$-CSF expression is under control of the $P_L$ promoter and gene N-ribosome binding site.

Figure 15 is an SDS gel showing expression of mG-CSF and $\nabla_1$-mG-CSF. The $\nabla_1$-mG-CSF mobilily reflects a slightly smaller size consistant with deletion of $NH_2$-terminal Met and Thr.

A. G-CSF Cloning and Expression

The G-CSF of the present invention was detected in a purified mRNA fraction obtained by sucrose density gradient centrifugation of induced MIA PaCa-2 cells as described in PCT Publication No. W086/04607 published August 14, 1986 and assigned to the assignee of the present invention.

mRNA fractions obtained from the gradient were injected into Xenopus laevis oocytes; the oocytes were incubated, and the supernatants tested for the ability to stimulate murine bone marrow proliferation (bmp). Peak bmp activity was found in supernatants from oocytes that had been injected with the 14, 15 and 16S MIA PaCa-2 mRNA fractions although peak hybridization to a CSF-1 specific probe was obtained with the 18S mRNA fraction. This phenominon is illustrated in Figure 1A which shows a plot of bmp activity and CSF hybridization intensity per mRNA fraction.

Further, investigation by the present inventors has determined that the peak bmp activity associated with the slower sedimenting RNA fractions is due to the activity of G-CSF as is shown in Figure 3A and 3B. These figures show that peak hybridization of a G-CSF specific probe with MIA PaCa-2 mRNA fractions sedimenting slower than 18S. Thus, the present invention encompasses an mRNA fraction of human cells that is capable of producing G-CSF in a host cell.

"G-CSF" as used herein means a protein having the effect of stimulating the production of primarily granulocyte colonies or granulocyte-macrophage colonies in a colony forming assay using bone marrow cell progenitors of an appropriate species. A protein having this activity has the deduced amino acid sequence shown in Figure 4 herein and is considered to be within the scope of the invention.

G-CSF according to the invention may be isolated from the MIA PaCa-2 cell line. In addition, as shown in Figure 2, G-CSF specific RNA sequences are clearly detectable in the mRNA of LD-1 cells and 5637 cells. Thus, G-CSF obtained by cloning from these cell lines as well as any other cell line which is G-CSF probe positive, is considered within the scope of the invention. Mature G-CSF produced by the LD-1 cell line has been isolated and amino-acid sequenced. 65% of the LD-1 produced protein has a thr residue at the $NH_2$-terminus and 35% has a pro residue at the $NH_2$-terminus.

As is the case for all proteins, the precise chemical structure of G-CSF depends on a number of factors. As ionizable amino and carboxyl groups are present in the molecule, a particular protein may be obtained as an acidic or basic salt, or in neutral form. All such prepartions which retain their activity when placed in suitable environmental conditions are included in the definition. Further, the primary amino acid sequence may be augmented by derivatization using sugar moieties (glycosylation) or by other supplementary molecules such as lipids, phosphate, acetyl groups and the like, more commonly by conjugation with saccharides. The primary amino acid structure may also aggregate to form complexes. Certain aspects of such augmentation are accomplished through post-translational processing systems of the producing host; other such modification may be introduced in vitro. In any event, such modifications are included in the definition so long as the activity of the protein, as defined above, is not destroyed. It is expected, of course, that such modifications may quantitatively or qualitatively affect the activity, either by enhancing or diminishing the activity of the protein in the various assays.

Further, individual amino acid residues in the chain may be modified by oxidation, reduction, or other derivatization, and the protein may be cleaved to obtain fragments which retain activity. Such alterations which do not destroy activity do not remove the protein sequence from the definition.

Modifications to the primary structure itself by deletion, addition, or alteration of the amino acids incorporated into the sequence during translation can be made without destroying the activity of the protein. Such substitutions or other alterations result in proteins having an amino acid sequence which falls within the definition of proteins "having an amino acid sequence substantially equivalent to that of G-CSF".

For convenience, the mature G-CSF protein amino acid sequence shown in Figure 4, deduced from the cDNA clone illustrated herein, is designated mG-CSF (mature G-CSF) beginning at the amino acid residue threonine designated +1. Figure 4 shows the presence of a 30 residue putative signal sequence, which is presumably cleaved upon secretion from mammalian cells; mG-CSF is represented by amino acids 1-174 shown in that figure. Specifically included in the definition of human G-CSF are muteins which monomers and dimers, if any, are G-CSF and related forms of G-CSF, designated by their differences from mG-CSF. G-CSF derived from other species may fit the definition of "human" G-CSF by virtue of its display of the requisite pattern of activity as set forth above with regard to human substrate.

Also for convenience, the amino acid sequence of G-CSF will be used as a reference and other sequences which are substantially equivalent to this in terms of G-CSF activity will be designated by referring to the sequence shown in Figure 4. The substitution of a particular amino acid will be noted by reference to the number of the amino acid residue which it replaced. Thus, for example, $ser_{60}$ G-CSF refers to the protein which has the sequence shown in Figure 4 except that the amino acid at position 60 is serine rather than proline. Deletions are noted by a $\nabla$ followed by the numer of amino acids deleted from the N-terminal sequence, or by the number of amino acids remaining when residues are deleted from the C-terminal sequence, when the number is followed by a minus sign. Thus, $\nabla_4$-G-CSF refers to G-CSF of Figure 4 wherein the first four amino acids from the N-terminus have been deleted; $\nabla_{130}$- refers to G-CSF wherein the last 44 amino acids following amino acid 130 have been deleted.

"Operably linked" or "in operable linkage" refers to juxtaposition such that the normal function of the components can be performed. Thus, a coding sequence "operably linked" to control sequences refers to a configuration wherein the coding sequence can be expressed under the control of these sequences.

"Control sequences" refers to DNA sequences necessary for the expresion of an operably linked coding sequence in a particular host organism. The control sequences which are suitable for procaryotes, for example, include a promoter, optionally an operator sequence, a RBS, and possibly, other as yet poorly understood, sequences. Eucaryotic cells are known to utilize promoters, polyadenylation signals, and enhancers.

"Expression system" refers to DNA sequences containing a desired coding sequence and control sequences in operable linkage, so that hosts transformed with these sequences are capable of producing the encoded proteins. In order to effect transformation, the expression system may be included on a vector; however, the relevant DNA may then also be integrated into the host chromosome.

As used herein "cell", "cell line", and "cell culture" are used interchangeable and all such designations include progeny. Thus "transformants" or "transformed cells" includes the primary subject cell and cultures derived therefrom without regard for the number of transfers. It is also understood that all progeny may not be precisely identical in DNA content, due to deliberate or inadvertent mutations. Mutant progeny which have the same functionality as screened for in the originally transformed cell, are included. Where distinct designations are intended, it will be clear from the context.

Control Sequences and Corresponding Hosts

Procaryotes most frequently are represented by various strains of *E. coli*. However, other microbial strains may also be used, such as bacilli, for example *Bacillus subtilis*, various species of *Pseudomonas*, or other bacterial strains. In such procaryotic systems, plasmid vectors which contain replication sites and control sequences derived from a species compatible with the host are used. For example, *E. coli* is typically transformed using derivatives of pBR322, a plasmid derived from an *E. coli* species by Bolivar, et al., *Gene*, *2*:95 (1977). pBR322 contains genes for ampicillin and tetracycline resistance, and thus provides additional markers which can be either retained or destroyed in constructing the desired vector. Commonly used procaryotic control sequences which are defined therein to include promoters for transcription initiation, optionally with an operator, along with RBS sequences, include such commonly used promoters as the beta-lactamase (penicillinase) and lactose (lac) promoter systems (Chang, et al., Nature (1977) 198:1056) and the tryptophan (Trp) promoter system (Goeddel, et al., Nucleic Acids Res. (1980) 8:4057) and the lambda derived $P_L$ promoter and N-gene ribosome binding site (Shimatake, et al., Nature (1981) 292:128), which has been made useful as a portable control cassette, as set forth in PCT Publication No. WO85/03522 published August 15, 1985. However, any available promoter system compatible with procaryotes can be used.

As described in detail hereinbelow, the native G-CSF sequence has not proved to be effective for the production of G-CSF in such prokaryotic microbial hosts at high levels. In the present invention, alterations have been carried out to the 5' end of the mG-CSF coding sequence. In addition to placing an ATG start codon at the 5' end of the DNA sequence encoding mG-CSF, a number of changes have been made in the nucleotide sequence thereof without changing the amino acid sequence of the mG-CSF protein encoded thereby.

These nucleotide changes encompass codons primarily encoding the first 10 amino acids of mG-CSF. At least one nucleotide in the third position of at least one of these codons has been changed from a deoxyriboguanidine or deoxyribocytosine to a deoxyriboadenine. These changes are made without altering the amino acid residue encoded by the codon, and furthermore, the resulting altered codon is one which is not necessarily host-preferred, at least when the host cell is *E. coli*. More preferably, the third position codon alteration is carried out on at least one of the first 10 or 11 codons of mG-CSF wherein the 5' amino acid sequence of mG-CSF remains ThrProLeuGlyProAlaSerSerLeuPro or MetThrProLeuGlyProAlaSerSerLeuPro, when the additional codon encoding an N-terminal Met is included.

More preferably, the above-described G to A or C to A codon alterations are carried out on at least one of the first 4 or 5 codons of mG-CSF wherein the 5' amino acid sequence of mG-CSF remains ThrProLeuGly or MetThrProLeuGly when the additional codon encoding an N-terminal methionine is included.

In a more preferred embodiment, the above-mentioned changes to the third position nucleotide of codons encoding the 5' end of mG-CSF are carried out to at least three of the 10 codons of the DNA sequence encoding the amino acid sequence ThrProLeuGlyProAlaSerSerLeuPro.

In an even more preferred embodiment, the above-mentioned changes to the third position nucleotide of codons encoding the 5' end of mG-CSF are carried out to at least three of the 4 codons of the DNA sequence encoding the amino acid sequence ThrProLeuGly.

When the above-mentioned changes are made in the 5' end of the DNA sequence encoding mG-CSF that is in an E. coli compatible expression vector in operable linkage with the Trp promoter, both steady state mRNA transcripts encoding mG-CSF and a protein fraction having mG-CSF activity are readily detectable by Northern blotting and Xenopus laevis oocyte assay, bone marrow proliferation, or granulocyte colony stimulation assays, respectively. By contrast, the native mG-CSF DNA sequence in otherwise operable linkage with the Trp promoter yields neither mRNA transcript nor detectable by Northern blot nor a protein fraction having G-CSF activity in the above-mentioned assays.

The native G-CSF DNA sequence in an expression vector in operable linkage with the $P_L$ promoter gene N-RBS, yields detectable steady state mRNA transcripts encoding G-CSF, but no protein fraction having mG-CSF. However, when the altered mG-CSF DNA sequence according to the invention is placed in the same vector under the same promoter RBS control, a protein fraction is produced having mG-CSF activity.

The invention also concerns muteins of G-CSF in which the N-terminal methionine is not present when G-CSF is produced from a recombinant host. In prokaryotic recombinant hosts such as E. coli. the translation initiation codon ATG encodes the amino acid methionine. As a result, the amino or $NH_2$-terminus of recombinant eukaryotic proteins produced in these recombinant hosts has a methionine residue that is generally not found in the protein when produced in the eukaryotic cell from which the DNA was initially

obtained. This superfluous $NH_2$-terminal methionine, however, can be processed and removed by certain exopeptidase enzymes, most particularly methionine amino peptidase or met amino peptidase. This enzyme although capable of removing $NH_2$-terminal amino acid residues, specifically does not remove the $NH_2$-terminal methionine with equal efficiency from all proteins. In particular, the efficiency of the enzyme varies depending upon the identity of the amino acid residue adjacent to the $NH_2$-terminal methionine.

The precise specificity of the enzyme in this regard cannot be determined without testing nearly an infinite number of substrates; however, a useful rule of thumb is set forth by Sherman, F., et al., Bio Assays 3:27-31 (1985). Sherman et al. based their considerations for specificity of Met-amino-peptidases on the observed forms of mutants of iso-1-cytochrome-C from yeast and the published primary sequence of 82 mature intracellular proteins. They conclude that methionine is usually cleaved from residues with a side chain having a radius of gyration of 1.29 A or less, but generally not cleaved from residues with side chains larger than 1.43 A. This is consistent with the observation that mutationally altered iso-1-cytochrome-C taken in consideration with other published sequences of other proteins from procaryotic and eucaryotic systems indicate that N-terminal methionine is cleaved when it precedes residues of alanine, cysteine, glycine, proline, serine, threonine, or valine, but not when it precedes residues of arginine, asparagine, aspartic acid, glutamine, glutamic acid, isoleucine, leucine, lysine, or methionine. These results are generally consistent with those set forth in the illustrations below. However, some exceptions occur where the radius of gyration for the second amino acid is higher than 1.29 A and the secondary and tertiary structure or other conditions are particularly favorable. Therefore, this aspect of the specificity of the enzyme is intended as a general guideline and it should be borne in mind that even the specificity of particular amino peptidase have not been determined with exact precision, i.e., not all possible tertiary structures have been tested. Therefore, in order to fall within the definition of "Met-aminopeptidase", the enzyme needs only to meet the requirement of specific cleavage of N-terminal methionine without cleavage of internal methionine residues and without cleavage of N-terminal residues other than methionine from any peptide.

Among the $NH_2$-terminal amino acid sequences that may be processed by amino terminal methionine peptidase, are those in which the methionine is followed by ala, gly or pro. In addition, $NH_2$-terminal methionine followed by ala-met, gly-met, ala-ser, gly-gly, ala-pro, or pro-thr is expected to be processed.

Thus, any of the form of G-CSF or muteins of G-CSF in which the above-mentioned sequences comprise the N-terminal portion of the amino acid will fall within the definition of G-CSF according to the invention so long as the biological activity of G-CSF is maintained. In particular, a deletion mutant of G-CSF, in which the threonine residue following the $NH_2$-terminal methionine is deleted to yield a $\nabla_1$-G-CSF is illustrated in some detail hereinbelow. Specifically mutated forms of G-CSF in which the $thr_1$ residue is substituted with ala, or gly, ala-ser, gly-gly, or in which the order of pro and thr are reversed will also fall within the scope of the invention. DNA species encoding the above-indicated muteins of G-CSF may be made by site specific mutagenesis of the cDNA encoding native G-CSF using known techniques. Oligonucleotides encoding these muteins may also be substituted for DNA sequences encoding the native G-CSF amino terminal amino acid sequence through ligation of oligodeoxyribonucleotides encoding the altered sequenced into specifically digested G-CSF DNA sequences.

B. General Methods for Carrying out the Invention

Transformations

Depending on the host cell used, transformation is done using standard techniques appropriate to such cells. The calcium treatment employing calcium chloride, as described by Cohen, S. N., Proc. Natl. Acad. Sci. (USA) (1972) 69:2110, or the $RbCl_2$ method described in Maniatis, et al., Molecular Cloning: A Laboratory Manual (1982) Cold Spring Harbor Press, p. 254 was used for procaryotes or other cells which contain substantial cell wall barriers. For mammalian cells without such cell walls, the calcium phosphate precipitation method of Graham and van der Eb, Virology (1978) 52:546 may be used or the method of Wang, et al., Science, 228:149 (1985). Transformations into yeast are carried out according to the method of Van Solingen, P., et al., J. Bact. (1977) 130:946 and Hsiao, C. L., et al., Proc. Natl. Acad. Sci. (USA) (1979) 76:3829.

## Screening cDNA Libraries

cDNA libraries are screened using the colony hybridization procedure. Lifts of colonies are made onto nitrocellulose filter papers (S & S type BA-85). The colonies are lysed and DNA fixed to the filter by treatment for five minutes with 0.5 M NaOH, 1.5 M NaCl, and are then washed twice for five minutes each time with 1.0 M Tris pH 8, 3 M NaCl. Filters are air dried and baked at 80°C for two hours. The duplicate filters are prehybridized at 45-50°C for one hour in 5 x SSC, 10 x Denhardt's solution (0.2% polyvinylpyrrolidone, 0.2% Ficoll, 0.2% BSA), 0.1% SDS, 50 mM sodium phosphate pH 7.0, and 100 μg/ml tRNA.

Hybridization of the filters is done in a solution similar to that described above for prehybridization, but also contains 10% dextran sulfate with kinased probe at $1 \times 10^6$ CPM/ml under conditions which depend on the stringency desired. Typical moderately stringent conditions employ temperatures of 45-50°C for 16-20 hours with 1-5 ml/filter of DNA hybridization buffer containing probe. For higher stringencies higher temperatures are employed. The filters are washed three times for 15 minutes each time at appropriate temperatures using 3 x SSC, 0.1% SDS, air dried, and are autoradiographed at -70°C for two to three days.

## Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercialy available restriction enzymes. See, e.g., New England Biolabs, Product Catalog. In general, about 1 μg of plasmid or DNA sequence is cleaved by one unit of enzyme in about 20 μl of buffer solutions. In the examples herein, typically, a 3-10 fold excess of restriction enzyme is used to insure complete digestion of the DNA substrate. Incubation times of about one hour to two hours at 37°C or other appropriate temperatures are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacylamide gel or agarose gel electrophoresis using standard techniques. A general description of size separations is found in Methods in Enzymology (1980) 65:499-560 or Maniatis, Molecular Cloning: A Laboratory Manual, Cold Spring Harbor Laboratory, Cold Spring Harbor, NY (1982).

Restriction cleaved fragments may be blunt ended by treating with the large fragment of E. coli DNA polymerase I (Klenow) in the presence of the four deoxynucleotide triphosphates (dXTPs) using incubation times of about 15 to 25 minutes at 20 to 25°C in 50 mM Tris pH 7.6, 50 mM NaCl, 6 mM $MgCl_2$, 6 mM DTT and 5-10 μM dXTPs. The Klenow fragment fills in at 5' overhangs but chews back protruding 3' single strands, even though the four dXTPs are present. If desired, selective repair can be performed by supplying only one of the, or selected, dXTPs within the limitations dictated by the nature of the sticky ends. After treatment with Klenow, the mixture is extracted with phenol/chloroform and ethanol precipitated followed by running over a Sephadex G-50 spin column. Treatment under appropriate

conditions with S1 nuclease results in hydrolysis of any single-stranded portion.

Synthetic oligonucleotides are prepared by the triester method of Matteucci, et al. (J. Am. Chem. Soc. (1981) 103:3185) or using commercially available automated oligonucleotide synthesizers. Kinasing of single strands prior to annealing or for labeling is achieved using an excess, e.g., approximately 10 units of polynucleotide kinase to 0.1 nmole substrate in the presence of 50 mM Tris, pH 7.6, 10 mM $MgCl_2$, 5 mM dithiothreitol, 1-2 mM ATP, 1.7 pmoles $\gamma$-32P-ATP (2.9 mCi/mmole), 0.1 mM spermidine, 0.1 mM EDTA.

Ligations are performed in 15-30 μl volumes under the following standard conditions and temperatures: 20 mM Tris-Cl pH 7.5, 10 mM $MgCl_2$, 10 mM DTT, 33 μg/ml BSA, 10 mM-50 mM NaCl, and either 40 μM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 μg/ml total DNA concentrations (5-100 nM total end concentration). Intermolecular "blunt end" ligations (usually employing a 10-30 fold molar excess of linkers) are performed at 1 μM total ends concentration.

In vector construction employing "vector fragments", the vector fragment is commonly treated with bacterial alkaline phosphatase (BAP) in order to remove the 5' phosphate and prevent religation of the vector. BAP digestions are conducted at pH 8 in approximately 150 mM Tris, in the presence of $Na^+$ and $Mg^{+2}$ using about 1 unit of BAP per μg of vector at 60°C for about one hour. Vector fragments subjected to this treatment are referred to herein as "bapped". In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

For portions of vectors derived from cDNA or genomic DNA which require sequence modifications, site specific primer directed mutagenesis is used. This is conducted using a synthetic oligonucleotide primer complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridizaton of an exact match, but at which the mismatches with the original strand are sufficient to prevent hybridization. Plaques which hybridize with the probe are then picked, cultured, and the DNA recovered. Details of site specific mutation procedures are described below in specific examples.

Verification of Construction

In the constructions set forth below, correct ligations for plasmid construction are confirmed by first transforming *E. coli* strain MM294 obtained from *E. coli* Genetic Stock Center, CGSC 6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al., Proc. Natl. Acad. Sci. (USA) (1969) 62:1159, optionally following chloramphenicol amplification (Clewell, D. B., J. Bacteriol. (1972) 110:667). The isolated DNA is analyzed by restriction enzyme mapping and/or sequenced by the dideoxy method of Sanger, F., et al., Proc. Natl. Acad. Sci. (USA) (1977) 74:5463 as further described by Messing, et al., Nucleic Acids Res. (1981) 9:309, or by the method of Maxam, et al., Methods in Enzymology (1980) 65:499.

Hosts Exemplified

Host strains used in cloning and expression herein are as follows:

For cloning and sequencing, and for expression of construction under control of most bacterial promoters, *E. coli* strain MM294 (supra), Talmadge, K., et al., Gene (1980) 12:235; Messelson, M., et al., Nature (1968) 217:1110, was used as the host. For expression under control of the $P_L$ N-RBS promoter, *E. coli* strain K12 MC1000 lambda lysogen, $N_7N_{53}$cI857Sus$P_{80}$, ATCC 39531 (hereinafter sometimes referred to as MC1000-39513 λDG95 or DG95) is used.

For M13 phage recombinants, *E. coli* strains susceptible to phage infection, such as *E. coli* K12 strain DG98 are employed. The DG98 strains has been deposited with ATCC July 13, 1984 and has accession number 1965.

In addition to bacteria, eucaryotic microbes, such as yeast, may also be used as hosts. Laboratory strains of *Saccharomyces cerevisiae*, Baker's yeast, are most used although a number of other strains are commonly available. While vectors employing the 2 micron origin of replication are illustrated, Broach, J. R., Meth. Enz. (1983) 101:307, other plasmid vectors suitable for yeast expression are known (see, for example, Stinchcomb, et al., Nature (1979) 282:39, Tschempe, et al., Gene (1980) 10:157 and Clarke, L., et al., Meth. Enz. (1983) 101:300). Control sequences for yeast vectors include promoters for the synthesis of glycolytic enzymes (Hess, et al., J. Adv. Enzyme Reg. (1968) 7:149; Holland, et al., Biochemistry (1978) 17:4900). Additional promoters known in the art include the promoter for 3-phosphoglycerate kinase (Hitzman, et al., J. Biol. Chem. (1980) 255:2073), and those for other glycolytic enzymes, such as glyceraldehyde-3-phosphate dehydrogenase, hexosekinase, pyruvate decarboxylase, phosphofructokinase, glucose-6-phosphate isomerase, 3-phosphoglycerate mutase, pyruvate kinase, triosphosphate isomerase, phosphoglucose isomerase, and glucokinase. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, ibid). Alterations to the 5' end of the mG-CSF DNA sequence analogous to those shown to be effective with the Trp promoter and $P_L$ promoter may also be carried out for use with such eukaryotic promoters.

It is also believed terminator sequences are desirable at the 3' end of the coding sequences. Such terminators are found in the 3' untranslated region following the coding sequences in yeast-derived genes. Many of the vectors illustrated contain control sequences derived from the enolase gene containing plasmid peno46 (Holland, M. J., et al., J. Biol. Chem. (1981) 256:1385) or the LEU2 gene obtained from YEp13 (Broach, J., et al., Gene (1978) 8:121), however any vector containing a yeast compatible promoter, origin of replciation and other control sequences is suitable.

It is also, of course, possible to express genes encoding polypeptides in eukaryotic host cell cultures derived from multicellular organisms as described further hereinbelow. See, generally for example, Tissue Culture, Academic Press, Cruz and Patterson, editors (1973). Useful host cell lines include murine myelomas N51, VERO and HeLa cells, COS-7 cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al., Nature (1978) 273:113), or other viral promoters such as those derived from polyoma, Adenovirus 2, bovine papiloma virus, or avian sarcoma viruses, or immunoglobin promoters and heat shock promoters. General aspects of mammalian cell host system transformations have been described by Axel, U.S. Patent No. 4,399,216 issued August 16, 1983. It now appears, also that "enhancer" regions are important in optimizing expression; these are, generally, sequences found upstream of the promoter region. Origins of replication may be obtained, if needed, from viral sources. However, integration into the chromosome is a common mechanism for DNA replication in eucaryotes. Plant cells are also now available as hosts, and control sequences compatible with plant cells such as the nopaline synthase promoter and polyandenylation signal sequences (Depicker,, A., et al., J. Mol. Appl. Gen. (1982) 1:561) are available.

The invention will be better illustrated in relationship to the following examples which are intended by the inventors to be exemplary and non-limiting.

## Example I

### Isolation and Expression of cDNA Encoding Human G-CSF

A cDNA clone encoding human granulocyte stimulating factor was isolated from the MIA PaCa-2 cell line, and was expressed using a recombinant vector in COS-7 cells. The MIA PaCa-2 cell line described in Yunis, A. A. et al., Experimental Hematol, 12:838-843 (1984), is an established cell line publically available from the Cell Repository Lines (CRL) collection of the American Type Culture Collection, 12301 Parklawn Avenue, Bethesda, MD 20895 under accession number ATCC CRL 1420. The cDNA clone was sequenced and the corresponding amino acid sequence was deduced.

### A. Initial Detection of mRNA From MIA PaCa-2 Cells Having High BMP Activity, But Low Hybridization to CSF-1 Probes

The human derived pancreatic carcinoma cell line MIA PaCa-2 was used as a source of mRNA to validate CSF-1 specific probes and for the formation of a cDNA library containing an intronless form of the human CSF-1 coding sequence. The MIA PaCa-2 cell line produces CSF-1 at a level approximately 10 fold below that of the murine L-929 cells.

Negative control mRNA was prepared from MIA PaCa-2 cells maintained in serum-free medium, i.e., under conditions wherein they do not produce CSF-1. Cells producing CSF-1 were obtained by reinducing CSF-1 production after removal of the serum.

Cells were grown to confluence in roller bottles using Dulbecco's Modified Eagles' Medium (DMEM) containing 10% fetal calf serum, and produce CSF-1 at 2000-6000 units/ml. The cell cultures were washed, and reincubated in serum-free condition to suppress CSF-1 formation. For negative controls, no detectable CSF-1 was produced after a day or two of incubation without serum. Reinduced cells were obtained by addition of phorbol myristic acetate (100 ng/ml) to obtain production after several days of 1000-2000 units/ml.

The MIA PaCa-2 mRNA was isolated by lysis of the cell in isotonic buffer with 0.5% NP-40 in the presence of ribonucleoside vanadyl complex (Berger, S. L., et al., Biochemistry (1979) 18:5143) followed by phenol/chloroform extraction, ethanol precipitation, and oligo dT chromatography, and an enriched mRNA preparation obtained. In more detail, cells are washed twice in PBS (phosphate buffered saline) and are resuspended in IHB (140 mM NaCl, 10 mM Tris, 1.5 mM $MgCl_2$, pH 8) containing 10 mM vanadyl adenosine complex (Berger, S. L., et. al., supra).

A non-ionic detergent of the ethylene oxide polymer type (NP-40) is added to 0.5% to lyse the cellular, but not nuclear membranes. Nuclei are removed by centrifugation at 1,000 x g for 10 minutes. The post-nuclear supernatant is added to two volumes of TE (10 mM Tris, 1 mM ethylenediaminetetraacetic acid (EDTA), pH 7.5) saturated phenol chloroform (1:1) and adjusted to 0.5% sodium dodecyl sulfate (SDS) and 10 mM EDTA. The supernatant is re-extracted four times and phase separated each time by centrifugation at 2,000 x g for 10 minutes. The RNA is precipiated by adjusting the sample to 0.25 M NaCl, adding 2 volumes of 100% ethanol and storing at -20°C. The RNA is pelleted at 5,000 x g for 30 minutes, is washed with 70% and 100% ethanol, and is then dried. Polyadenylated ($polyA^+$) messenger RNA (mRNA) is obtained from the total cytoplasmic RNA by chromatography on oligo dT cellulose (Aviv, J., et al., Proc. Natl. Acad. Sci. (1972) 69:1408-1412). The RNA is dissolved in ETS (10 mM Tris, 1 mM EDTA, 0.5% SDS, pH 7.5) at a concentration of 2 mg/ml. This solution is heated to 65°C for five minutes, then quickly chilled to 4°C. After bringing the RNA solution to room temperature, it is adjusted to 0.4 M NaCl and is slowly passed through an oligo dT cellulose column previously equilibrated with binding buffer (500 mM NaCl, 10 mM Tris, 1 mM EDTA, pH 7.5 0.5% SDS). The flow-through is passed over the column twice more. The column is then washed with 10 volumes of binding buffer. Poly$A^+$ mRNA is eluted with aliquots of ETS, extracted once with TE-saturated phenol chloroform and is precipitated by the addition of NaCl to 0.2 M and 2 volumes of 100% ethanol. The RNA is reprecipitated twice, is washed once in 70% and then in 100% ethanol prior to drying.

Total mRNA was subjected to 5-20% by weight sucrose gradient centrifugation in 10 mM Tris HCl, pH 7.4, 1 mM EDTA, and 0.5% SDS using a Beckman SW40 rotor at 20°C and 27,000 rpm for 17 hours. The mRNA fractions were then recovered from the gradient by ethanol precipitation, and injected into *Xenopus laevis* oocytes in the standard translation assay. The oocyte products of the RNA fractions were assayed in the bone marrow proliferation assay of Moore, R. N. et al., J. Immunol. (1983) 131:2374, and of Prystowsky, M. B., et al., Am. J. Pathol. (1984) 114:149 and the fractions themselves were assayed by dot blot hybridization to a 32-mer probe corresponding to the DNA in the second exon of the genomic sequence (exon II probe). These results are summarized in Figure 1A and B.

The broken line in Figure 1A shows the response in the bone marrow proliferation assay of the supernatants from the *Xenopus laevis* oocytes; Figure 1B shows the dot blot results. The most strongly hybridizing fraction, 11, corresponds to greater than 18S, while the most active fractions 8 and 9 correspond to 14-16S.

The mRNA was also fractionated on a denaturing formaldehyde gel, transferred to nitrocellulose, and probed with exon II probe. Several distinct species ranging in size from 1.5 kb to 4.5 kb were found, even under stringent hybridization conditions.

B. Isolation G-CSF mRNA From MIA PaCa-2 Cells

Confluent MIA PaCa-2 cells were stimulated in serum free Dulbecco's minimum essential medium (DMEM) for 4 days with phorbol myristate acetate (50 ng/ml) and retinoic acid (10 μM). The RNA was prepared as described by Chirguin et al.; briefly, the cells were lysed in 5 M guanidine isothiocyanate followed by centrifugation through a 5.7 M cesium chloride (CsCl) cushion; poly $A^+$ RNA was prepared from the lysed cells by one selection cycle on oligo (dT) cellulose as described in Maniatis et al., supra.

In more detail, cells were lysed in a solution which contained 5 M guanidine isothiocyanate, 0.025 M Na-citrate, pH 7, 0.5% sarcosine and 8% β-mercaptoethanol. Molecular biology grade CsCl was made up to 5.7 M or 40% w/v and buffered with 0.02 M Tris pH 7.5 and 0.002 M Na-EDTA. All solutions were prepared under RNase free conditions and passed through 0.45 μ Millipore filters before use. The lysed cells were then layered onto SW28 ultracentrifuge tubes which contained layers of 10 ml 5.7 M CsCl and 6 ml 40% CsCl.

After centrifugation at 26,000 rpm for 18 hours, the RNA pellet was dissolved in $dH_2O$ and ethanol precipitated twice. Polyadenylated ($PolyA^+$) messenger RNA (mRNA) was obtained by chromatography of the total RNA on oligo (dT) cellulose as described in Maniatis, supra (at page 197).

The RNA is dissolved in sterile $H_2O$ and heated to 65°C for five minutes. An equal volume of a solution containing 0.040 M Tris Cl pH 7.6 1.0 M NaCl, 0.002 M EDTA and 0.2% SDS is quickly added and the sample is cooled to room temperature. The sample is then loaded on an oligo (dT) column that has been equilibrated with a buffer containing 0.020 M Tris pH 7.6, 0.5 M NaCl, 0.001 M EDTA and 0.1% SDS. The flow through is collected, heated to 65°C, cooled to room temperature and passed over the column once more. The column is then washed with 10 volumes of wash buffer (0.02 Tris, 0.1 M NaCl, 0.001 M EDTA, 0.1% SDS). Poly $A^+$ mRNA is eluted with aliquots of 0.01 M Tris pH 7.5, 0.001 M EDTA and ethanol-precipitated twice.

334 μg of MIA PaCa-2 Poly $A^+$ mRNA was fractionated to 5-25% by weight sucrose gradient centrifugation in 20 mM Tris HCl, pH 7.5, 1 mM EDTA, and 0.5% sarcosine using a Beckman SW40 rotor at 20°C and 27,800 rpm for 16 hours. The mRNA fractions were collected in 400 μl

fractions and ethanol precipitated twice. Fractions were pooled and resuspended in 15 µl $H_2O$.

Northern blots were prepared by the electrophoresis of 5 µg of poly $A^+$ RNA per lane or 1 µl of each pooled RNA fraction through 1% agarose gels containing 0.5 M formaldehyde followed by blotting onto nitrocellulose filters. After baking the filters for 1.5 hours at 80°C, they were prehybridized (5 x SSC, 10 x Denhardt's solution (0.2% polyvinylpyrrolidone, 0.2% Ficoll, 0.2% BSA), 0.1% SDS, 50 mM sodium phosphate pH 7.0, and 100 µg/ml tRNA) for one hour at 55°C. The blots were hybridized for 16 hours at 55°C in a similar solution that also contained 10% dextran sulfate and $10^6$ cpm per ml of an oligonucleotide probe labeled with $\gamma^{32}P$-ATP and polynucleotide kinase. The blot shown in Figure 3B was washed at 55°C in 3 x SSC, 0.1% SDS. This oligonucleotide probe had the sequence 5'-GTAGGTGGCACACAGCTTCTCCTG-3' and was designed based on the sequence of the CHU-2 cDNA clone described by Nagata et al., Nature, 319:415-418 (1986).

The RNA pools that were described in the above assay were translated in a *Xenopus laevis* oocytes assay by the injection of 50 nl of of RNA into each oocyte as described in Gurdon et al., Nature, 233:177-180 (1971). Supernatants of 10 µl per oocyte were collected after 40 hours and assayed for CSF activity.

The *Xenopus laevis* supernatants from each hybridization fraction were assayed for CSF activity in a murine bone marrow cell proliferation assay as described in Moore, et al., J. Immunol., 131:2374-2378 (1983). Briefly, in this assay, 5 x $10^4$ murine bone marrow cells per/well were incubated in 96-well plates (12 x 8) with serially diluted *Xenopus laevis* oocyte supernatants made from positively hybridizing mRNA fractions. After three days, $^3H$ thymidine (0.5 µCi/well) was added, and after six hours the cells were harvested and counted in a liquid scintillation counter.

Peak bone marrow proliferation was found in *Xenopus laevis* oocyte supernatants made with mRNA fractions that were most strongly positive in Northern blots with the above-described oligonucleotide probe as shown in Figure 3.

C. Identification of G-CSF Clones in a MIA PaCa-2 cDNA Library

A cDNA library was prepared from the enriched MIA PaCa-2 mRNA as described in Kawasaki, et al., Science, 30:291-296 (1985). Briefly, the method used oligo (dT) priming of the poly $A^+$ tails and AMV reverse transcriptase employing the method of Okayama, H. et al., Mol. Cell Biol., 3:280-289 (1983) This method results in a higher proportion of full length clones than does poly (dG) tailing and effectively uses as host vector portions of two vectors therein described, and readily obtainable from the authors, pcDV1 and pL1. The resulting vectors contain the insert between vector fragments containing proximal BamHI and XhoI restriction sites; the vector contains the pBR322 origin of replication, and ampicillin resistance gene and SV40 control elements which result in the ability of the vector to effect expression of the inserted sequences in COS-7 cells.

A $1.2 \times 10^6$ clone library in E. coli obtained from the above enriched MIA PaCa-2 mRNA by the Okayama and Berg method was then probed using the same oligonucleotide probe that yielded a positive signal on the most active pooled MIA PaCa-2 mRNA fractions. To probe the library, E. coli containing the Okayama-Berg vectors were grown up on nutrient medium. Colonies were lifted onto nitrocellulose filter papers and were lysed. DNA was fixed to the filter by treatment for five minutes with 0.5 mM NaOH, 1.5 M NaCl. Filters were then washed twice for five minutes each time with 1.5 M Tris pH 8, 3 M NaCl and were air dried and baked at 80°C for two hours.

The filters for the screening were prehybridized and hybridized to the $\gamma^{32}P$ labeled probe as described above in Section B of the example, but both prehybridization and hybridization were carried out at 50°C. Plasmids pP12 and pP28 were determined to be probe positive and were further characterized.

D. Sequencing of G-CSF MIA PaCa-2 Plasmids cDNA

pP12 was digested with BamHI and subcloned into a M13mp19 vector and sequenced using the dideoxy chain-termination method.

The DNA sequence and predicted protein sequence of pP12 are shown in Figure 4. The cDNA insert in pP12 is 1510 base pairs long excluding the poly (dG) and poly (dA) tails; it contains 11 more bases of 5' untranslated sequence than the CHU-2 G-CSF clone. The major difference between this clone derived from MIA PaCa-2 and CHU-2 cDNA clones of Nagata et al. is a 9 base pair insertion (GTGATGGAG) in the CHU-2 clone that would encode the amino acid residues Val-Ser-Glu just prior to cys-36 in the MIA PaCa-2 G-CSF as indicated by the arrow in Figure 4. There are two other differences; an A at position 588 in the MIA PaCa-2 clone (G in the CHU-2 clone) is a silent third base change, and a T at position 1237 in the MIA PaCa-2 clone (C in the CHU-2 clone) is in the 3' untranslated region.

E. <u>Activity of the pP12 and pP28 Proteins Produced in COS-7 Cells</u>

Plasmids pP12 and pP28 were purified using a CsCl gradient and COS-7 cells were transfected using a modification (Wang, A. M., et al., <u>Science</u>, <u>228</u>:149 (1985)) of the calcium phosphate coprecipitation technique. After incubation for three days, supernatants were collected and G-CSF production was assayed in a colony forming assay and the murine bone marrow proliferation assay, as described above.

CSF activity was measured by both murine bone marrow cell proliferation and colony formation assays. For the colony assay, 1 x $10^5$ mononuclear bone marrow cells from Balb/c mice were used in a total of 1 ml, containing 5% cell supernatant, 15% fetal calf serum, and 0.3% agar in RPMI 1640 medium. Colonies of greater than 40 cells were counted after seven days, and cell type determined by cytocentrifuging individual colonies and staining with a modified Wright stain. Each unit of CSF stimulates the formation of one colony. For a control, COS-7 cells were mock transfected under the same conditions by carrying the cells through the transfection steps without exposure to a plasmid. The results of the assay are described below in Table I.

TABLE I

Production of CSF Activity by Transfected COS-7 Cells

| Transfection | Proliferation (cpm) | Colony Assay Units/ml | %G[a] | %GM[b] |
|---|---|---|---|---|
| pP12 | 4595 | >280 | 58 | 42 |
| pP28 | 6594 | >280 | 83 | 17 |
| Mock | 633 | 0 | 0 | 0 |

a = Colonies containing only granulocytes.

b = Colonies containing both granulocytes and macrophages.

## EXAMPLE II

### Cloning G-CSF for Expression in E. coli

#### A. Preparation of G-CSF Sequence:Insertion of 5' HindIII and ATG Codon

In order to facilitate manipulation of the G-CSF DNA sequence in various cloning and expression vectors, the G-CSF sequence was altered by inserting a HindIII site and ATG codon in mG-CSF 3' of the GCC codon encoding alanine (-1).

Plasmid pP12 was digested with BamHI endonuclease and the digest was electrophoresed in a Tris-acetate, 0.5% low-melting point agarose gel. The small fragment band carrying the G-CSF DNA sequence was melted and ligated into BamHI-digested M13mp19 using T4 DNA ligase under sticky end conditions. After ligation, the mixture was used to transform *E. coli* strain DG98, ATCC accession number 39,768. The transformed cells were plated in the presence of 0.3 mM isopropylthiogalactoside (IPTG) obtained from Sigma Chemicals (St. Louis, MO) and 0.3 mM X-gal on a lawn of DG98 and grown at 37°C. Non-alpha complementary white plaques were grown in liquid broth and a sample of the culture was used to purify replicative form (RF) DNA. The presence of the insert was confirmed by BamHI digestion and sizing the fragment on a 0.7% agarose gal. Phage having the expected insert were designated pA3.

A HindIII site and ATG initiation codon were placed in the G-CSF sequence adjacent to and in frame with the codon encoding the N-terminal threonine of the mG-CSF sequence by means of site-specific mutagenesis using a chemically synthesized purified 33-mer oligodeoxyribonucleotide having the following sequence:

5'-GTG CAG GAA GCC AAG CTT ATG ACC CCC CTG GGC-3'.

The HindIII site immediately upstream of the ATG codon permits the convenient cloning of the mG-CSF-encoding DNA sequence in subsequent manipulation. Approximately 10 pmoles (pM) of the 33-mer was hybridized to about 1 pM of single stranded DNA from pA3 in about 13 µl of 10 mM Tris pH 7.4, 90 mM NaCl, 10 mM $MgCl_2$ by heating to 85°C for five minutes, followed by 45°C for 20 minutes. The annealed mixture was chilled on ice and adjusted to 18 µl by the addition of dithiothrietol to 10 mM, each dXTP to 0.5 mM and 5 units of DNA Polymerase I Klenow fragment. The reaction mixture was incubated on ice for 20 minutes followed by incubation at room temperature for one hour. The repair reaction mixture was then used to transform E. coli strain DG98 as above, plated onto agar plates and incubated overnight to obtain phage plaques.

Plaques were blotted using nitrocellulose filter circles and the filters were treated as described to lyse the cells, denature the DNA, neutralize, rinse, fix the DNA to the filter and incubate in prehybridization buffer. The 33-mer oligonucleotide was end labeled with $\gamma^{32}P$ using polynucleotide kinase and hybridized to the filters at 45°C overnight. Filters were washed and subjected to autoradiography. Probe positive plaques were grown in liquid culture and characterized by BamHI HindIII digestion and agarose gel sizing to confirm the presence of the expected fragments. One clone having the desired insert was designated pApooL. pApooL was digested with BamHI and HindIII. The approximately 1500 base pair BamHI-HindIII fragment was isolated, purified, and retained for ligation into a plasmid having the desired promoter and terminator sequences.

B. Placement of G-CSF Coding Sequence Under Trp Control

To place the G-CSF coding sequence under Trp control and under the influence of the BT positive retroregulatory element (PRE), the G-CSF coding sequence was cloned into plasmid pAW703. pAW703 is a plasmid having the Trp promoter and PRE in operable linkage with a HindIII-BamHI fragment which contains the gene encoding human tumor necrosis factor (TNF).

As described below in detail, pAW703 is digested with BamHI and HindIII and the BamHI and HindIII fragment of pApool encoding G-CSF is ligated in place of the TNF fragment.

The following describes the construction of pAW703 and the cloning of G-CSF into this plasmid to make pPD1. An alternate construction using pTrp3 is also described.

B.1. Construction of pAW703

Plasmid pAW703 was made from plasmids pAW701 and pAW711 as follows. Plasmid pAW711 (ATCC 39,918) which contains a 400 bp BamHI-SalI fragment encoding the PRE was digested with SalI and BamHI endonucleases under conditions suggested by the supplier to yield an approximately 400 bp fragment containing the PRE. The digest was loaded on a 1% agarose preparatory gel and the small SalI-BamHI fragment containing the PRE was isolated, electroeluted and retained.

Plasmid pAW701 was used to obtain the Trp control sequences for regulating expression of the gene.

pAW701, a derivative of plasmid pTrp3, was made by digesting pTrp3 with BamHI and HindIII endonucleases and ligation of the large HindIII-BamHI fragment with HindIII-BamHI fragment carrying the TNF gene. In the construction of pAW703, pAW701 was digested with BamHI and SalI endonucleases. The large BamHI-SalI fragment of pAW701 containing the Trp promoter HindIII site and TNF sequence was isolated by electroelution from a preparative agarose gel, and was ligated to the small BamHI-SalI fragment containing the PRE, under sticky end conditions using T4 DNA ligase to produce pAW703.

Both pAW703 and pApool were digested with BamHI and HindIII under conditions suggested by the manufacturer. The large HindIII-BamHI fragment from pApooL was gel isolated and ligated with the pAW703 digest using T4 DNA ligase.

The ligation mixture was used to transform competent *E. coli* strain MM294. Transformants were screened for ampicillin resistance. Plasmids from a number of ampicillin resistant colonies were isolated, digested with HindIII and BamHI and run on a 1.5% agarose gel with DNA sizing standards. One clone having the expected size fragments was designated pPD1.

B.2. Trp Expression Vectors

pTrp3 is a host expression vector containing a Trp promoter and RBS immediately 5' of a HindIII restriction site, thus permitting insertion of a coding sequence having a HindIII site immediately 5' of a start codon under control of the Trp promoter. The backbone vector for pTrp3 is pBR322. pTrp3 was deposited with the American Type Culture Collection, 12301 Parklawn Drive, Rockville, MD (ATCC) on December 18, 1984, and has accession number 39,946.

B.2.A. Preparation of pTrp3

To construct the host vector containing the Trp control sequences behind a HindIII site, the Trp promoter/operator/ribosome binding site sequence, lacking the attenuator region, was obtained from pVH153, obtained from C. Yanofsky, Stanford University. Trp sequences are available in a variety of such plasmids known in the art. pVH153 was treated with HhaI (which cuts leaving an exposed 3' sticky end just 5' of the Trp promoter) blunt ended with Klenow, and partially digested with TaqI. The 99 bp fragment corresponding to restriction at the TaqI site, 6 nucleotides preceding the ATG start codon of Trp leader were isolated, and then ligated to EcoRI (repair)/ClaI digested, pBR322 to provide pTrp3.

An improved form of pTrp3 also includes a positive retroregulatory sequence in a position in the vector downstream of the coding sequence to be expressed as described hereinbelow.

An expression vector for G-CSF having the same control sequences as pAW703 may be made directly from pTrp3 (ATTC 39,940) and pAW711 (ATCC 39,918) and pP12 as follows.

pTrp3 is digested with HindIII and SalI. The large HindIII-SalI fragment is isolated by electroelution from a preparative gel. The small BamHI-SalI fragment containing the PRE is isolated from pAW711 as described above and ligated to the large HindIII-SalI fragment of pTrp3 under sticky end conditions with T4 DNA ligase. pPD1 may also be made directly by combining the BamHI-HindIII fragment of pP12 with the above-mentioned fragments from pTrp3 and pAW711 and ligating under sticky end conditions with T4 DNA ligase.

C. Placing a BamHI Restriction Site Downstream of the G-CSF Translation Termination Site

The DNA sequence encoding G-CSF in pA3 contained approximately 930 nucleotides in the transcribed but untranslated region 3' of the translation stop codon TGA in the G-CSF sequence. In order to remove these untranslated codons and to place transcription termination and mRNA stability under the influence of the positive retroregulatory element, a BamHI site was placed by site-specific mutagenesis immediately 3' of the G-CSF translation termination signal.

A chemically synthesized purified 27-mer deoxyribonucleotide was chemically synthesized using a commercial DNA synthesizer and had the following sequence:

CCC AGC CCT GAG GAT CCA AGC CCT CCC

Approximately 10 pM of the 27-mer was hybridized to about 1 pM of single-stranded DNA from PpA3 in about 13 µl of 10 mM Tris pH 7.4, 90 mM NaCl, 10 mM $MgCl_2$ by heating to 85°C for five minutes, followed by 45°C for 20 minutes. The annealed mixture was chilled on ice and adjusted to 18 µl by the addition of dithiothreitol to 10 mM, each dXTP to 0.5 mM and 5 units of DNA Polymerase I Klenow fragment. The reaction mixture was incubated on ice for 20 minutes, followed by incubation at room temperature for about one hour.

The reaction mixture was then used to transform *E. coli* strain DG98, plated onto agar plates and incubated overnight to obtain phage plaques. Plaques were blotted using nitrocellulose filter circles and the filters were treated as described to lyse the cells, denature the DNA, neutralize, rinse, fix the DNA to the filter and incubate in prehybridization buffer. The 27-mer oligonucleotide was end labeled with $^{32}\gamma$ phosphate using polynucleotide kinase and hybridized to the filters at 45°C overnight. Filters were washed and subjected to autoradiography. One of the probe positive plaques was designated pBpool.

pBpool was grown in liquid culture and digested with *ApaI* and *BamHI* endonucleases under conditions specified by the manufacturer. The coding region of G-CSF has an internal *ApaI* site. This digestion generated an *ApaI*-*BamHI* fragment of approximately 540 base pairs, with the newly inverted *BamHI* end immediately 3' of the translation stop codon of the mG-CSF gene.

Plasmid pPD1 was also digested with *ApaI* and *BamHI* under conditions suggested by the supplier. This digestion yielded an *ApaI*-*BamHI* fragment in which the *ApaI* site was the same as that described above and the *BamHI* site is 3' of the untranslated region of the G-CSF gene.

The two digests were mixed at a molar excess of digested pBpool to pPD1 and ligated under sticky end conditions using T4 DNA ligase. The ligation mixture was transformed into *E. coli* strain MM294. Transformants were screened for ampicillin resistance. Several colonies were isolated and digested with *HindIII* and *BamHI*. A colony having the correct length *HindIII*-*BamHI* insert was designated pPD2.

D. Placing mG-CSF Under $P_L$ Control

Plasmid pLAPHβ is a plasmid having the $P_L$ promoter and gene N-RBS controlling the expression of the Aphβ gene. The Aphβ gene is contained in a *HindIII*-*BamHI* fragment. The plasmid further contains the positive retroregulatory element 3' to the Aphβ gene with a *BamHI*

site directly 5' of the PRE. Plasmid pLAphβ was made from plasmid pFC54.T (ATCC number 39,739). Plasmid pLAPHβ is exactly the same as pFC54.T except that the latter plasmid has a HindIII-BamHI fragment encoding des-alanyl-IL-2 in place of the Aphβ encoding fragment. Thus, the following steps to place the expression of G-CSF under temperature sensitive $P_L$ promoter-gene N-RBS and PRE control can also be carried out using pFC54.T. The HindIII-BamHI fragment of pPD2 having the ATG initiation codon was placed under $P_L$ promoter control and PRE influence as follows:

Plasmid pLAPHβ was digested with HindIII and BamHI under conditions suggested by the supplier. Plasmid pPD2 was digested with the same endonucleases. The digests were combined and ligated with T4 DNA ligase with the pPD2:pLAPHB digests at a 10:1 molar ratio.

After ligation, the ligation mixture was used to transform competent E. coli host strain λNDG95. Ampicillin resistant colonies were selected and plasmids were isolated therefrom, digested with BamHI and HindIII and a transformant having the expected insert of approximately 540 bp was designated pJD1.

## EXAMPLE III

### Expression of mG-CSF in pPD2 and pJD1

#### A. Growth and Induction of pPD2 in Transformed E. coli Strains MM294 and pJD1 in Transformed E. coli in λN DG95

Initial attempts to produce mG-CSF using the two different expression vectors were not successful. The first vector, pJD1, (Figure 5) consisted of the DNA encoding the mature mG-CSF protein under the control of the bacteriophage λ $P_L$ promoter and the λ gene N-RBS. The Bacillus thuringiensis positive retroregulating element (PRE) is immediately downstream of the mG-CSF coding region. In addition, the origin of replication, derived from ColE1, contained mutations that conferred a temperature-sensitive $Cop^-$ phenotype. E. coli cells, strain DG95λ (λ $N_7N_{33}$ C1837 sus P80), harboring pJD1 were grown at 30°C to an optical density of 0.3 at 600 nm. The $P_L$ promoter and plasmid replication were then induced by shifting the temperature to 42°C for three hours.

The second vector pPD2, consisted of DNA encoding the mG-CSF protein under the control of the E. coli Trp promoter. The Bacillus thuringiensis positive retroregulating element was immediately downstream of the mG-CSF coding region. E. coli cells, strain MM294, were grown overnight in M9 medium plus 100 μg/ml of Trp and 50 μg/ml of ampicillin. In the morning the cells were washed twice with M9 medium and the Trp promoter was then induced by setting up cultures at an optical density of 0.05 at 600 nm in M9 medium without Trp. The cells were grown at 37°C to an optical density of 0.8 at 600 nm.

After induction of pJD1 and pPD2, extracts of induced and uninduced cells were prepared by boiling the cells for five minutes in Laemmli loading buffer (Laemmli, Nature, 227:680-685 (1970)) which contains sodium dodecyl sulfate (SDS) and 2-mercaptoethanol (2ME). After electrophoresis through 12.5% polyacrylamide gels (PAGE), containing SDS and 2ME, and staining with Coomassie brilliant blue, an induced protein band of the molecular weight corresponding to mG-CSF could not be detected.

B. Steady State mG-CSF mRNA Level After Induction of Cells Harboring pJD1 and pPD2

To determine the steady level of G-CSF transcripts in cells harboring pJD1 and pPD2, RNA was prepared as follows: 20 ml of induced or uninduced cells was mixed with 20 ml of ice cold phosphate buffered saline. The cells were pelleted by centrifugation at 4°C and resuspended in 3 ml of 0.15 M sucrose-20 mM sodium acetate. 0.3 ml of a 20 mg/ml lysozyme solution was added and the mixture was incubated on ice. 0.15 ml of 20% SDS was added and the mixture was extracted with equal volumes of phenol:chloroform (1:1) until the interface was clear (about 4 times). After two ethanol precipitations, the nucleic acid mixture was resuspended in 100 μl of 20 mM Tris (pH 7.5)-10 mM $MgCl_2$. Two microliters of a 1 mg/ml solution of RNase-free DNase I was added and the mixture was incubated at 37°C for 10 minutes. Five μl of 20% SDS was added and the samples were then phenol:chloroform (1:1) extracted twice and ethanol precipitated 2 times.

The Northern blotting analysis was performed as described using a modification of a published protocol (Maniatis et al., Molecular Cloning:A Laboratory Manual, Cold Spring Harbor, N.Y. (1982)). Briefly, Northern blots were prepared by the electrophoresis of RNA through 1% agarose gels containing 0.5 M formaldehyde followed by blotting onto nitrocellulose filters. After baking the filters for 1.5 hours at 80°C, they were prehybridized for one hour at 45°C in: 5 x SSC, 0.2% polyvinylpyrrolidone, 0.2% Ficoll, 0.2% bovine serum albumin, 0.1% SDS, 50 mM sodium phosphate pH 7.0, and 100 μg/ml tRNA. The blots were hybridized for 16 hours at 45°C in a similar solution that also contained dextran sulfate (10%) and $10^6$ cpm per ml of the mG-CSF specific oligonucleotide 5'-CGCTGCGCCATCGCCCTGGATCTT-3' that had been labeled with $\gamma^{32}P$ ATP and polynucleotide kinase. The blots were washed in 3.2 M tetramethylammonium chloride for 15 minutes at 62°C. The bands corresponding to mG-CSF mRNA were then detected by autoradiography.

Figure 10 shows that induced cells harboring pJD1 contained a mG-CSF mRNA of the expected size as well as smaller mG-CSF related mRNA while cells harboring pPD2 did not contain detectable mG-CSF mRNA.

## EXAMPLE IV

A. Construction of a $P_L$ Vector with Alternative mG-CSF Codons

Two oligodeoxyribonucleotides were chemically synthesized and purified and had the following sequences: A = 5'-AGCTTATGACACCATTAGGAC-3', B = 5'-TAATGGTGTCATA-3'.

DNA of pJD1 was digested with the restriction enzymes HindIII and ApaI and, after electrophoresis, the large fragment was isolated from a Tris-acetate, 0.5% low-melting-point agarose gel (Crease et al., Methods in Enzymology, 101:78 (1983)). This fragment was mixed with 20 pmole of each of the above two oligonucleotides. This mixture was ligated with T4 DNA ligase, and used to transform competent *E. coli* cells of the strain DG95. The cells were plated on agar plates containing ampicillin and colonies containing recombinant plasmids (pJD4A and pJD4B) were identified by colony hybridization (Maniatis et al. supra) using oligonucleotide A which had been labeled with $\gamma^{32}P$ ATP and polynucleotide kinase. As shown in Figure 11, pJD4A and pJD4B, both encode the same protein as pJD1; however, codons 2, 3, and 4 of pJD4A and pJD4B are different from the ones used in pJD1. In addition, codon 5 in pJD4B also differs from pJD1. This difference between pJD4A and pJD4B results from the fact that the end of the oligomer duplex that was ligated to the ApaI site contained a single mismatch to the single-stranded end generated by the enzyme ApaI. This type of mismatch will not interfere with ligations (Hung and Wensink, Nucleic Acids Research, 12:1863-1874 (1984)); however, depending on the repair of this mismatch, some of the resulting clones will regenerate the ApaI site and have a pJD1-like 5th codon and some will lose the ApaI site and have a pJD4B-like 5th codon. The pJD4A and pJD4B constructions were verified by DNA sequence analysis.

B. Expression of mG-CSF Protein by pJD4A and pJD4B

*E. coli* harboring either pJD4A or pJD4B were induced and analyzed by SDS-PAGE as described for pJD1 in Example III. Figure 12 shows that an inducible protein, with a molecular weight corresponding to that of mG-CSF, is present in *E. coli* cells that harbor either pJD4A or pJD4B.

C. Construction of a $P_L$ Vector With Alternative Codons Encoding an Amino Methionine Peptidase Processable Amino Terminus of MG-CSF

Two oligonucleotides were chemically synthesized and purified and had the following sequences:

C: 5'AGCTTATGCCATTAGGAC3'
D: 3'   ATACGGTAAT   5'

DNA of plasmid pJD1 was digested with restriction enzymes HindIII and ApaI and the large fragment was isolated as described in IV.A. above. 20 pM each of oligonucleotides C and D were mixed with the large fragment, ligated with $T_4$ DNA ligase under sticky end conditions, and the ligated mixture was used to transform competent *E. coli* strain DG95. Cells were plated on agar containing ampicillin and Amp resistant colonies containing a recombinant plasmid were identified by hybridization with oligonucleotide C, which had been labeled with $\gamma^{32}P$ ATP using polynucleotide kinase. The plasmid obtained from the probe positive colonies was designated pJD6 and encoded a G-CSF species in which the $thr_1$ is deleted, i.e., $\nabla_1$-mG-CSF. The correct sequence was confirmed by dideoxy sequencing.

D. Expression of mG-CSF Protein by pPD6

*E. coli* DG95 harboring pPD6 was induced and analysed as described for pJD1 in Example III.

Figure 15 is a picture of an SDS-PAGE gel of proteins produced by *E. coli* harboring pPD6 and pJD5. The cell clearly shows that pPD6 produce an inducable protein with a slightly smaller molecular weight than MG-CSF produced by pJD5.

The protein band produced by pPD6 was isolated and purified from a large scale induction of pPD6. The purified protein was sequenced using an automated amino acid sequencer and showed that the $NH_2$-terminus of this protein is pro rather than met or thr.

EXAMPLE V

A. Construction of a Trp Expression Vector Containing Alternative mG-CSF Codons

DNA from pJD4A was digested with the restriction enzymes HindIII and BamHI. The small fragment, containing the coding region for mG-CSF, was isolated from Tris-acetate 0.5% low-melting-point agarose gel. DNA from pPD2 was digested with the restriction enzymes HindIII and BamHI and the large fragment was isolated from a Tris-acetate 0.5% low-melting-point agarose gel. These two fragments were ligated together with T4 DNA ligase and used to transform competent *E. coli* of the strain MM294. An ampicillin resistant colony containing the recombinant was picked and the construct was verified by DNA sequence analysis. The resulting plasmid was designated pPD5.

B. Expression of mG-CSF in pPD5

*E. coli* harboring pPD5 was induced and analyzed by SDS-PAGE as described for pPD2 in the previous example. Figure 13 shows that an inducible protein, with a molecular weight corresponding to that of mG-CSF, was present in *E. coli* cells that harbor pPD5.

C. Using Alternative mG-CSF Codons Increases the Steady State Level of mG-CSF mRNA

The steady state level of mG-CSF mRNA in induced cells harboring either pPD2 or pPD5 was compared by Northern analysis as described above. Figure 14 shows that induced cells harboring pPD5 contain a mG-CSF transcript of the expected size, while no mG-CSF transcripts were detected in induced cells harboring pPD2.

The following plasmids have been deposited in the American Type Culture Collection, Rockville, MD USA:

| Plasmid | Strain | Date | Accession # |
|---|---|---|---|
| pJD4A | *E. coli* K12 DG95λ | 8/12/86 | 67,181 |
| pJD4B | *E. coli* K12 DG95λ | 8/12/86 | 67,183 |
| pPD5A | *E. coli* K12 MM294 | 8/12/86 | 67,182 |
| pAW11 | *E. coli* K12 DG95λ | 11/8/84 | 39,918 |
| pTrp3 | *E. coli* MC1000 | 12/18/84 | 39,946 |
| pFC54.t | *E. coli* DG95λ | 8/7/84 | 39,789 |

These deposits were made under the provisions of the Budapest Treaty on the International Recognition of the Deposit of Microorganisms for the Purposes of Patent Procedure and the Regulations thereunder (Budapest Treaty). This assures maintenance of a viable culture for 30 years from date of deposit. The deposits will be made available by ATCC under the terms of the Budapest Treaty, and subject to an agreement between applicants and ATCC which assures permanent and unrestricted availability upon issuance of the pertinent U.S. patent. The Assignee herein agrees that if the culture on deposit die or be lost or destroyed when cultivated under suitable conditions, it will be promptly replaced upon notification with a viable speciment of the same culture. Availability of the deposits is not be be construed as a license to practice the invention in contravention of the rights granted under the authority of any government in accordance with its patent laws.

The invention includes the use of G-CSF as defined above or a mutein as defined above in preparing a medicament, e.g. in preparing a pharmaceutical or veterinary formulation.

In another aspect the invention includes a pharmaceutical or veterinary formulation comprising G-CSF as defined above or a mutein as defined above formulated for pharmaceutical or veterinary use respectively, optionally the formulation being in unit dosage form and/or also comprising an acceptable diluent, carrier or excipient.

The inventors have disclosed G-CSF and means for the efficient expression thereof. Variations within the scope of the invention may be made by those ordinarily skilled in the art without departing from the essence of the invention as claimed herein. The illustrative embodiments given hereinbefore are not limiting.

CLAIMS:

1. A modified DNA sequence encoding G-CSF, said G-CSF having the effect of stimulating the production of primarily granulocyte or granulocyte-macrophage colonies in colony forming assay using bone marrow cell progenitors of an appropritate species, wherein the 5' region thereof comprises deoxyriboadenine substituted for the deoxyriboguanine or deoxyribocytosine in the third position of at least one codon wherein said substitution does not change the amino acid sequence of G-CSF.

2. The DNA sequence according to claim 1 wherein said 5' region comprises a DNA sequence encoding the amino acid sequence Thr Pro Leu Gly or Thr Pro Leu Gly Pro Ala Ser Ser Leu Pro.

3. The DNA sequence according to claim 2 wherein at least 3 codons have said substitution.

4. The DNA sequence according to claims 1, 2 or 3 further comprising deoxyribothymidine substituted for deoxyribocytosine in the first position of at least one codon.

5. A modified DNA sequence encoding mature G-CSF wherein the 5' region thereof comprises codons selected from the group consisting of ACA CCA TTA GGA, ACA CCA TTA, ATG ACA CCA TTA GGA and ATG ACA CCA TTA.

6. A modified DNA sequence encoding a mutein of G-CSF, said mutein of G-CSF having the effect of stimulating the production of primarily granulocyte or granulocyte-macrophage colonies in colony forming assays using bone marrow cell progenitors of an appropriate species, wherein the mutein does not have an $NH_2$-terminal methionine when produced from a recombinant host.

7. The modified DNA sequence encoding a mutein of G-CSF of claim 6 wherein the $NH_2$-terminal end of said mutein encodes a sequence that is processed by met amino peptidase.

8. The modified DNA sequence encoding a mutein of G-CSF of claim 7 wherein the DNA sequence encodes an amino terminal end selected from the group consisting of met-ala, met-gly, met-pro, met-ala-met, met-gly-met, met-ala-ser, met-ala-pro, met-pro-thr, and met-pro-leu.

9. An expression vector comprising the DNA sequence of claims 1 to 8 in operable linkage with a promoter and ribosome binding site.

10. The expression vector of claim 9 in operable linkage with a promoter-ribosome binding site selected from the group consisting of $P_L$ promoter and ribosome binding sites operable therewith and Trp promoter-ribosome binding site.

11. An **E. coli** host transformed with the expression vector of claim 9 or claim 10.

12. A culture of **E. coli** transformed with the expression vector of claim 10 having a G-CSF content of at least about 3% of total cell protein to about 10% of total cell protein.

13. Human recombinant G-CSF.

14. An mRNA fraction isolated from human cells that encodes human G-CSF.

15. The expression vector of claim 10 comprising pJD4 or pPD5.

16. A mutein of G-CSF, said mutein of G-CSF having the effect of stimulating the production of primarily granulocyte or granulocyte-macrophage colonies in colony forming assays using bone marrow cell progenitors of an appropriate species wherein said mutein does not have an $NH_2$-terminal methionine.

17. The mutein of claim 16 wherein the $NH_2$-terminal end of said mutein has an amino acid sequence that is processed by met amino peptidase in a recombinant host.

18. The mutein of G-CSF of claim 17 wherein the amino terminal end is selected from the group consisting of met-ala, met-gly, met-pro, met-ala-met, met-gly-met, met-ala-ser, met-ala-pro, met-pro-thr and met-pro-leu.

19. The mutein of G-CSF of claim 18 which yields $\nabla_1$-mG-CSF when expressed in a recombinant host.

20. A mutein of mG-CSF selected from the group consisting of $ala_1$-mG-CSF, $gly_1$-mG-CSF, $pro_1$-mG-CSF, $pro_1$-$thr_2$-mG-CSF, $ala_1$-$met_2$-mG-CSF, $gly_1$-$met_2$-mG-CSF, $ala_1$-$ser_2$-mG-CSF, $ala_1$-$pro_2$-mG-CSF, $pro_1$-$leu_2$-mG-CSF and $\nabla_1$-mG-CSF.

21. $\nabla_1$-mG-CSF.

22. A method of producing a modified DNA sequence encoding G-CSF, said G-CSF having the effect of stimulating the production of primarily granulocyte or granulocyte-macrophage colonies in colony forming assay using bone marrow cell progenitors of an appropriate species, which method comprises substituting deeoxyriboadenine at the 5' region of the sequence for the deoxyriboguanine or deoxyribocytosine in the third position of at least one codon wherein said substitution does not change the amino acid sequence of G-CSF.

23. A method according to claim 22 and further defined by the specific feature(s) of any one or more of claims 2 to 4.

24. A method of making a modified DNA sequence encoding mature G-CSF which includes selecting codons for the 5' region thereof from the group consisting of ACA CCA TTA GGA, ACA CCA TTA, ATG ACA CCA TTA GGA and ATG ACA CCA TTA.

25. A method of producing a modified DNA sequence encoding a mutein of G-CSF, said mutein of G-CSF having the effect of stimulating the production of primarily granulocyte or granulocyte-macrophage colonies in colony forming assays using bone marrow cell progenitors of an appropriate species, wherein the mutein does not have an $NH_2$-terminal methionine when produced from a recombinant host, comprising modifying the $NH_2$-terminal sequence encoding portion of a DNA sequence encoding G-CSF, for example by subjecting cDNA encoding native G-CSF to site specific mutagenesis to produce an $NH_2$-terminal sequence wherein the $NH_2$-terminal methionine is susceptible to enzymatic cleavage or ligating an oligonucleotide encoding such an $NH_2$-terminal sequence to a specifically digested G-CSF DNA sequence.

26. A method according to claim 25 and further defined by the specific feature of claim 7 or of claim 8.

27. A process for preparing an expression vector which comprises forming an operable linkage between a promoter and ribosome binding site and a DNA sequence as defined in any one of claims 1 to 8, the promoter-ribosome binding site preferably being selected from the group consisting of $P_L$ promoter and ribosome binding sites operable therewith and Trp promoter-ribosome binding site.

28. A method of making an E. coli cell capable of expressing G-CSF which comprises transforming an E. coli host with an expression vector as defined in claim 9, claim 10 or claim 15.

29. A process for the preparation of recombinant G-CSF which comprises culturing an E. coli transformed host as defined in claim 11 or a culture as defined in claim 12 or E. coli cells made by the method of claim 28 so as to permit expression of the E. coli genome.

30. A process according to claim 29 wherein the G-CSF prepared thereby is as defined in any one of claims 16 to 21.

31. A pharmaceutical or veterinary formulation comprising G-CSF as defined in claim 13 or a mutein as defined in any one of claims 16 to 21 formulated for pharmaceutical or veterinary use respectively, optionally the formulation being in unit dosage form and/or also comprising an acceptable diluent, carrier or excipient.

32. The use of G-CSF as defined in claim 13 or a mutein as defined in any one of claims 16 to 21 in preparing a medicament, e.g. in preparing a pharmaceutical or veterinary formulation as defined in claim 31.

FIG. 1A

MIA-PACA GRADIENT BM PROLIF. ASSAY

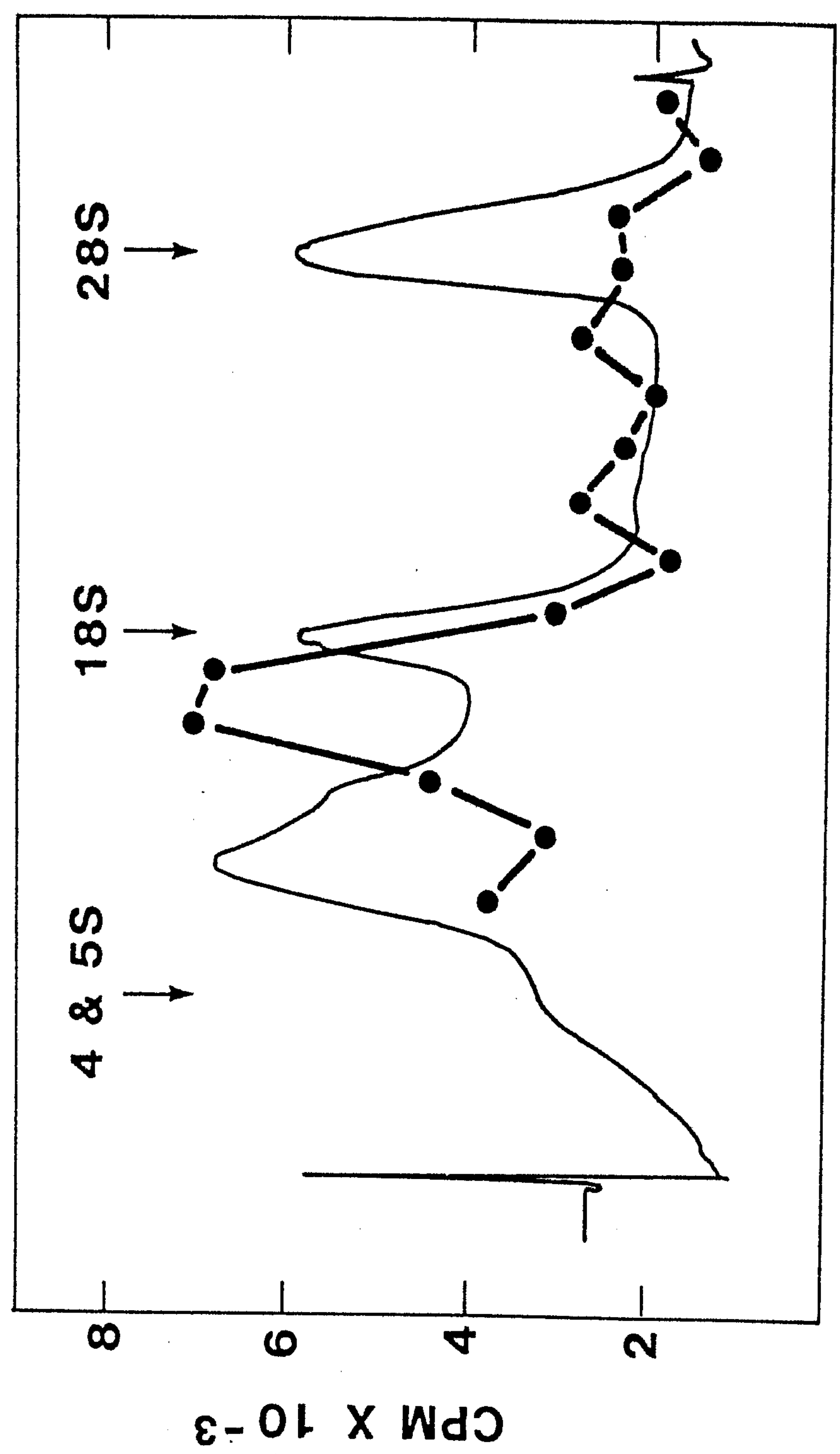

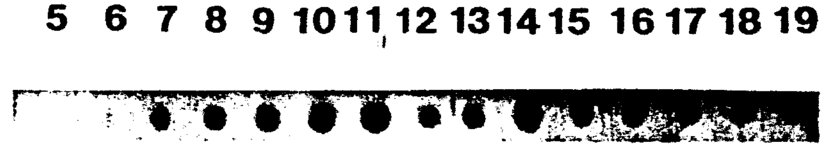


FIG. 1B

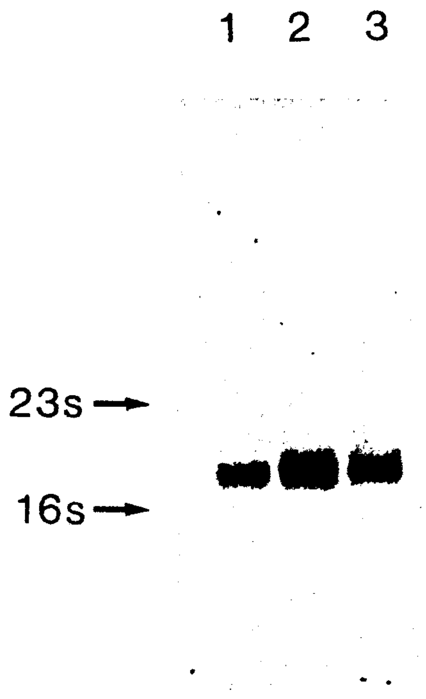


FIG. 2A

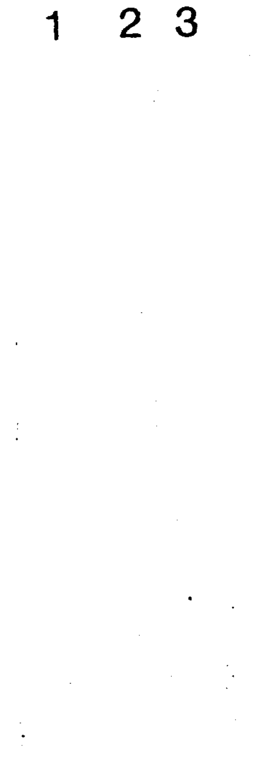


FIG. 2B

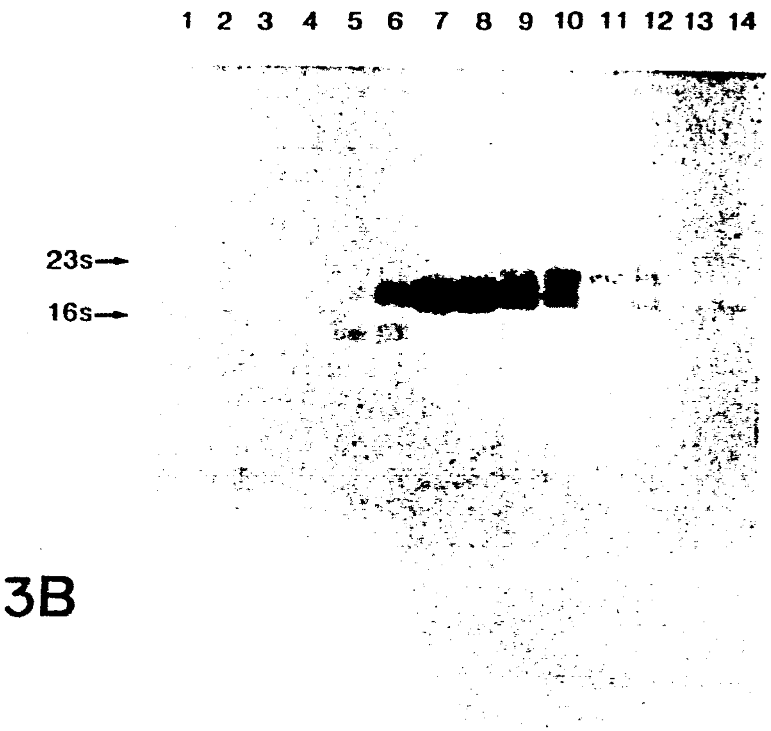


FIG. 3B

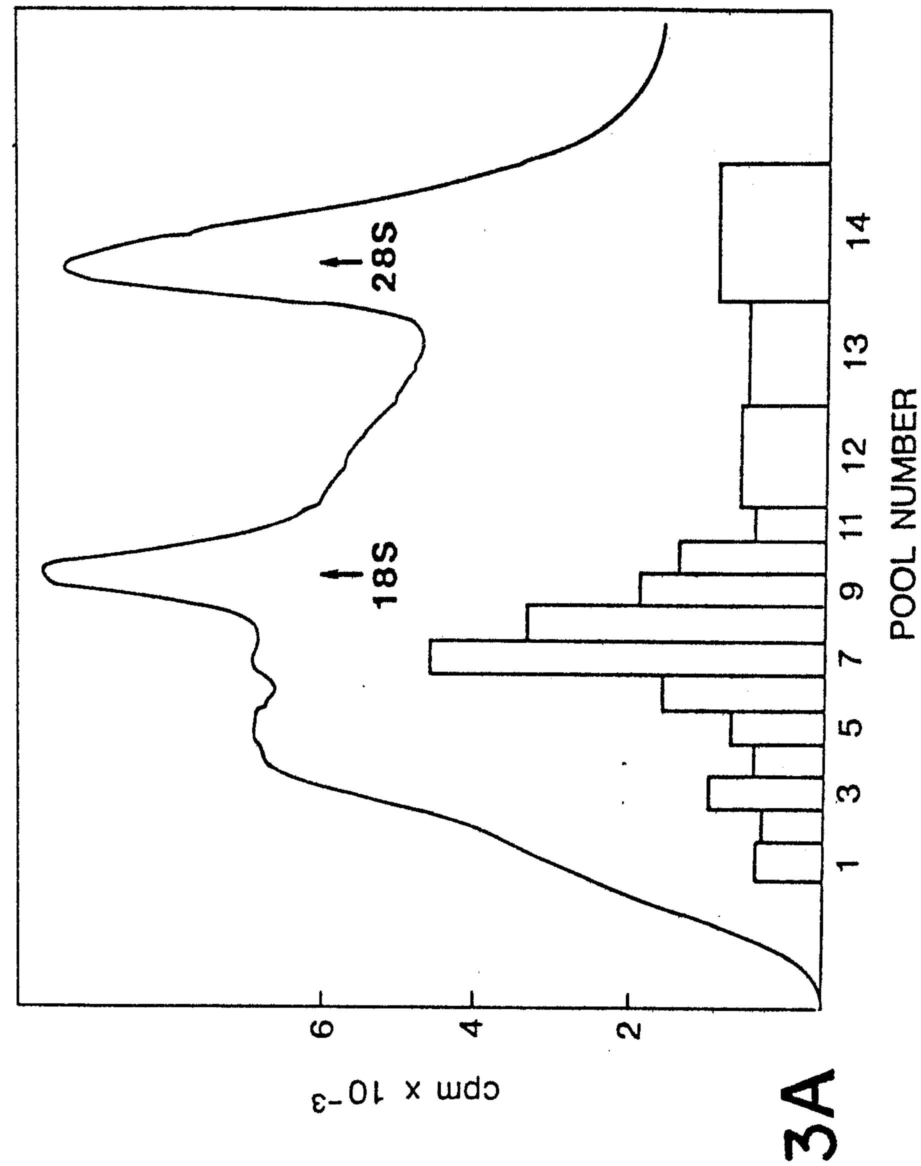


FIG. 3A

# FIG. 4

```
1    AAAAAACAGCCCGGAGCCTGCAGCCCAGCCCCACCCAGACCCATGGCTGGACCTGCCACC
                                               MetAlaGlyProAlaThr
                                                   -30
61   CAGAGCCCCATGAAGCTGATGGCCCTGCAGCTGCTGCTGTGGCACAGTGCACTCTGGACA
     GlnSerProMetLysLeuMetAlaLeuGlnLeuLeuLeuTrpHisSerAlaLeuTrpThr
              -20                            -10

121  GTGCAGGAAGCCACCCCCCTGGGCCCTGCCAGCTCCCTGCCCCAGAGCTTCCTGCTCAAG
     ValGlnGluAlaThrProLeuGlyProAlaSerSerLeuProGlnSerPheLeuLeuLys
                -1  1                        10

181  TGCTTAGAGCAAGTGAGGAAGATCCAGGGCGATGGCGCAGCGCTCCAGGAGAAGCTGTGT
     CysLeuGluGlnValArgLysIleGlnGlyAspGlyAlaAlaLeuGlnGluLysLeuCys
                20                           30

241  GCCACCTACAAGCTGTGCCACCCCGAGGAGCTGGTGCTGCTCGGACACTCTCTGGGCATC
     AlaThrTyrLysLeuCysHisProGluGluLeuValLeuLeuGlyHisSerLeuGlyIle
                40                           50

301  CCCTGGGCTCCCCTGAGCAGCTGCCCCAGCCAGGCCCTGCAGCTGGCAGGCTGCTTGAGC
     ProTrpAlaProLeuSerSerCysProSerGlnAlaLeuGlnLeuAlaGlyCysLeuSer
                60                           70

361  CAACTCCATAGCGGCCTTTTCCTCTACCAGGGGCTCCTGCAGGCCCTGGAAGGGATCTCC
     GlnLeuHisSerGlyLeuPheLeuTyrGlnGlyLeuLeuGlnAlaLeuGluGlyIleSer
                80                           90

421  CCCGAGTTGGGTCCCACCTTGGACACACTGCAGCTGGACGTCGCCGACTTTGCCACCACC
     ProGluLeuGlyProThrLeuAspThrLeuGlnLeuAspValAlaAspPheAlaThrThr
               100                          110

481  ATCTGGCAGCAGATGGAAGAACTGGGAATGGCCCCTGCCCTGCAGCCCACCCAGGGTGCC
     IleTrpGlnGlnMetGluGluLeuGlyMetAlaProAlaLeuGlnProThrGlnGlyAla
               120                          130

541  ATGCCGGCCTTCGCCTCTGCTTTCCAGCGCCGGGCAGGAGGGGTCCTAGTTGCCTCCCAT
     MetProAlaPheAlaSerAlaPheGlnArgArgAlaGlyGlyValLeuValAlaSerHis
               140                          150

601  CTGCAGAGCTTCCTGGAGGTGTCGTACCGCGTTCTACGCCACCTTGCCCAGCCCTGAGCC
     LeuGlnSerPheLeuGluValSerTyrArgValLeuArgHisLeuAlaGlnPro
               160                          170
661  AAGCCCTCCCCATCCCATGTATTTATCTCTATTTAATATTTATGTCTATTTAAGCCTCAT
721  ATTTAAAGACAGGGAAGAGCAGAACGGAGCCCCAGGCCTCTGTGTCCTTCCCTGCATTTC
781  TGAGTTTCATTCTCCTGCCTGTAGCAGTGAGAAAAAGCTCCTGTCCTCCCATCCCCTGGA
841  CTGGGAGGTAGATAGGTAAATACCAAGTATTTATTACTATGACTGCTCCCCAGCCCTGGC
901  TCTGCAATGGGCACTGGGATGAGCCGCTGTGAGCCCCTGGTCCTGAGGGTCCCCACCTGG
961  GACCCTTGAGAGTATCAGGTCTCCCACGTGGGAGACAAGAAATCCCTGTTTAATATTTAA
1021 ACAGCAGTGTTCCCCATCTGGGTCCTTGCACCCCTCACTCTGGCCTCAGCCGACTGCACA
1081 GCGGCCCCTGCATCCCCTTGGCTGTGAGGCCCCTGGACAAGCAGAGGTGGCCAGAGCTGG
1141 GAGGCATGGCCCTGGGGTCCCACGAATTTGCTGGGGAATCTCGTTTTTCTTCTTAAGACT
1201 TTTGGGACATGGTTTGACTCCCGAACATCACCGACGTGTCTCCTGTTTTTCTGGGTGGCC
1261 TCGGGACACCTGCCCTGCCCCCACGAGGGTCAGGACTGTGACTCTTTTTAGGGCCAGGCA
1321 GGTGCCTGGACATTTGCCTTGCTGGACGGGGACTGGGGATGTGGGAGGGAGCAGACAGGA
1381 GGAATCATGTCAGGCCTGTGTGTGAAAGGAAGCTCCACTGTCACCCTCCACCTCTTCACC
1441 CCCCACTCACCAGTGTCCCCTCCACTGTCACATTGTAACTGAACTTCAGGATAATAAAGT
1501 GTTTGCCTCCAAAAAAAAAAAAAAA
```

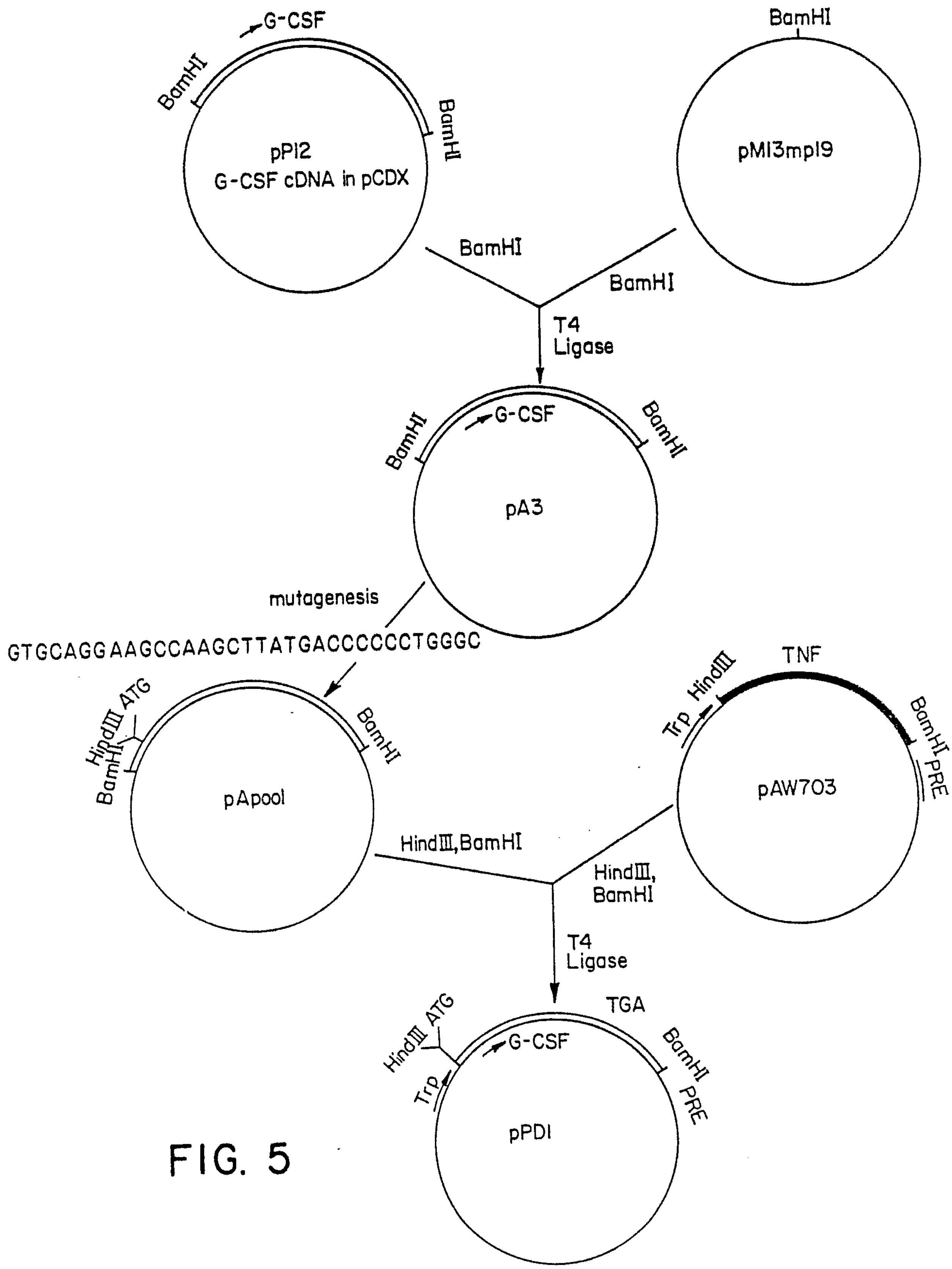
G-CSF
BamHI
BamHI
pPI2
G-CSF cDNA in pCDX
BamHI
pMI3mpI9
BamHI
BamHI
T4
Ligase
G-CSF
BamHI
BamHI
pA3
mutagenesis
GTGCAGGAAGCCAAGCTTATGACCCCCCTGGGC
HindIII ATG
BamHI
BamHI
pApool
TNF
Trp HindIII
BamHI
PRE
pAW703
HindIII, BamHI
HindIII,
BamHI
T4
Ligase
TGA
HindIII ATG
G-CSF
BamHI
Trp
PRE
pPDI

FIG. 5

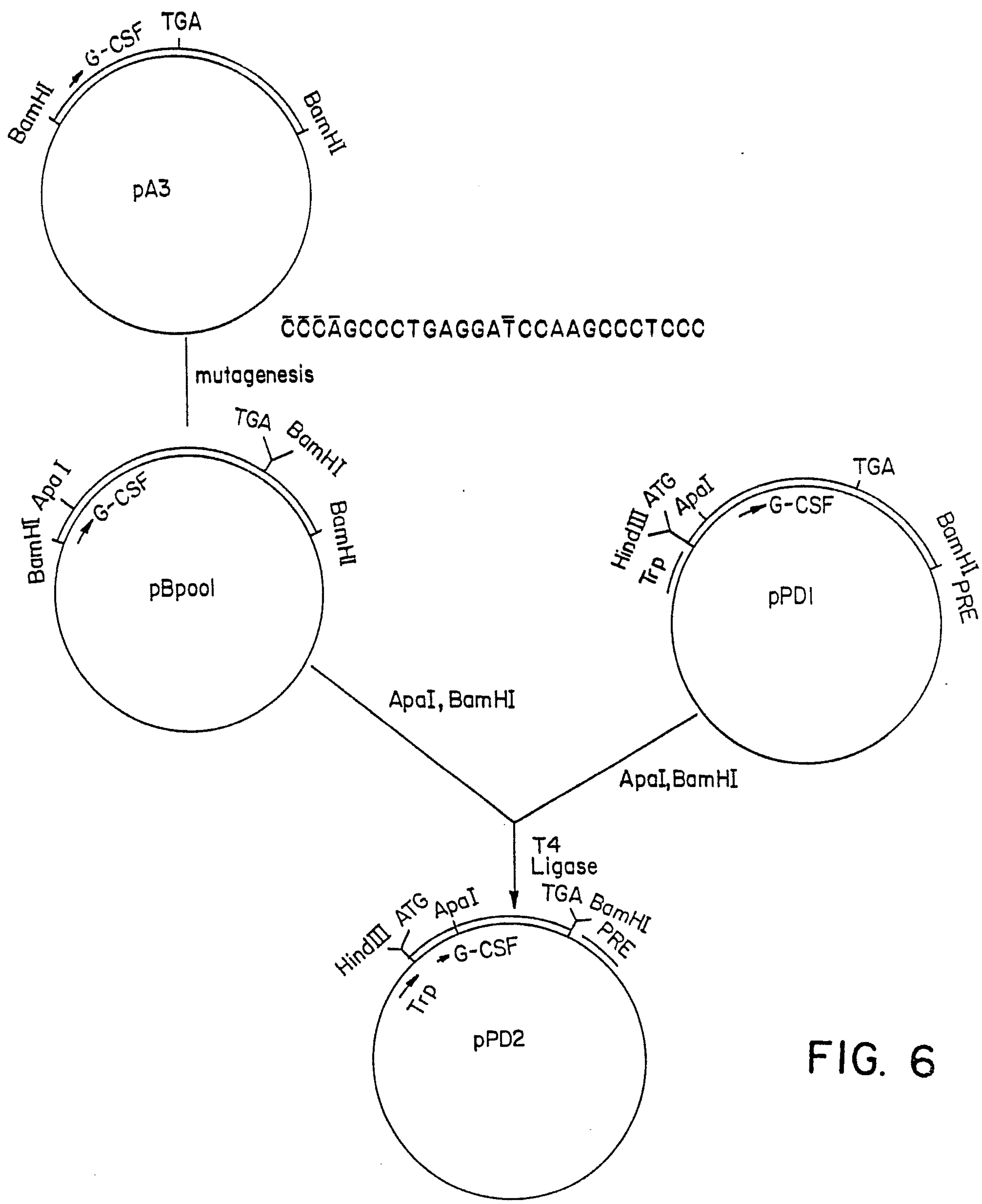
TGA
G-CSF
BamHI
BamHI
pA3
C̄C̄C̄ĀGCCCTGAGGAT̄CCAAGCCCTCCC
mutagenesis
TGA
BamHI
ApaI
G-CSF
BamHI
BamHI
pBpool
TGA
HindIII
ATG
ApaI
G-CSF
Trp
BamHI
PRE
pPD1
ApaI, BamHI
ApaI, BamHI
T4
Ligase
HindIII
ATG
ApaI
TGA
BamHI
PRE
G-CSF
Trp
pPD2

FIG. 6

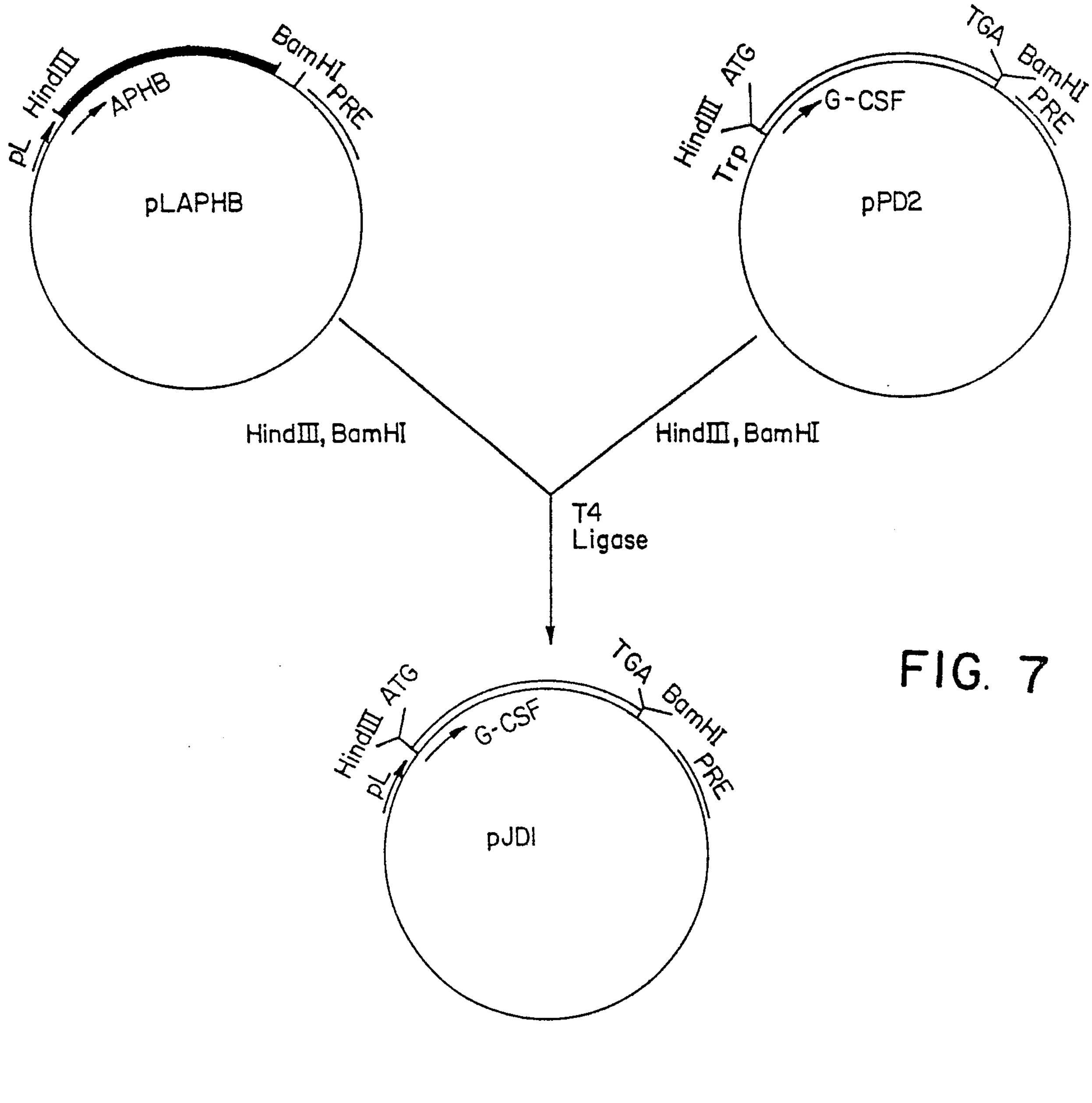
pL
HindIII
APHB
BamHI
PRE
pLAPHB
HindIII ATG
Trp
G-CSF
TGA
BamHI
PRE
pPD2
HindIII, BamHI
HindIII, BamHI
T4
Ligase
HindIII ATG
pL
G-CSF
TGA
BamHI
PRE
pJDI

FIG. 7

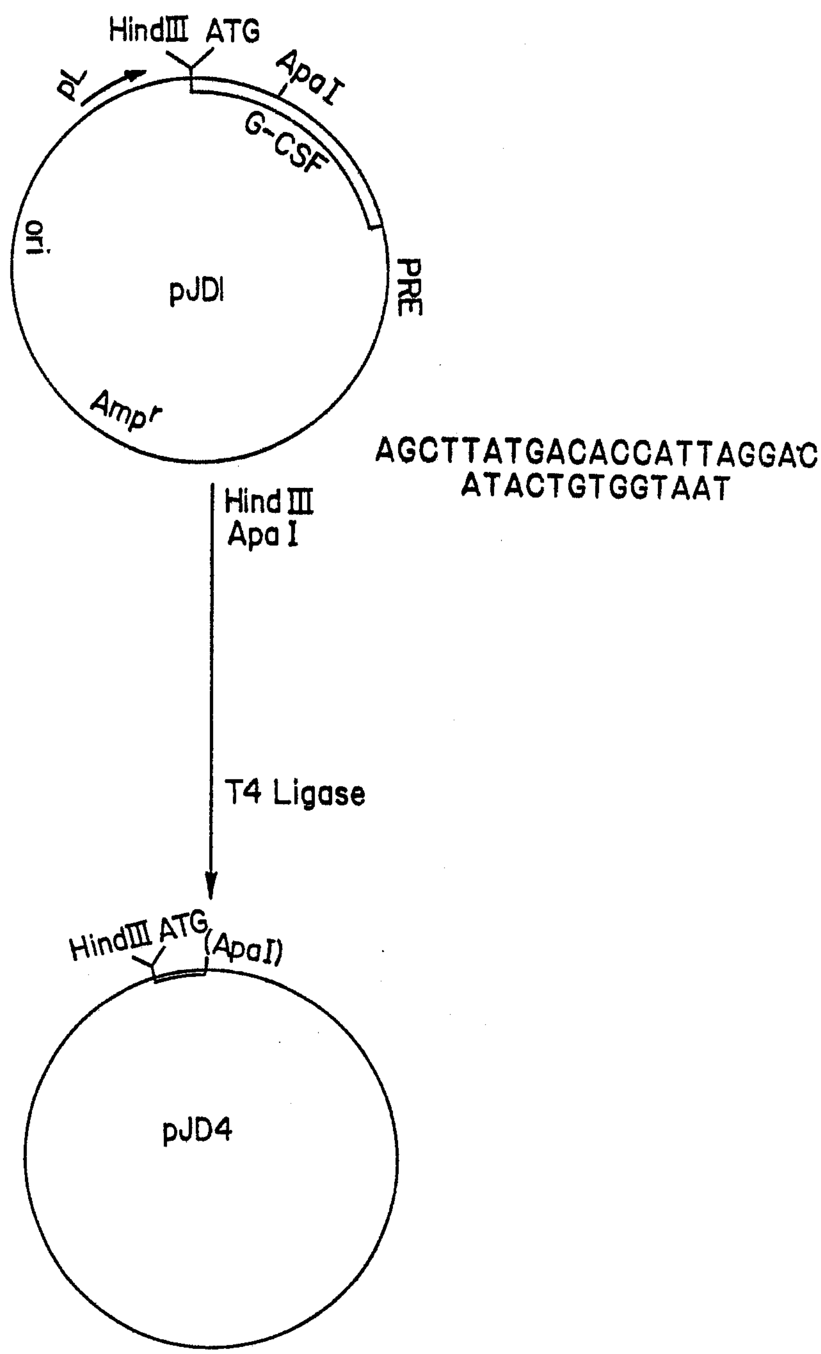


FIG. 8

| | | |
|---|---|---|
| | Apa I | |
| ATG ACC CCC CTG GGC CCT | | pJDI PARENT |
| A AT A A | | pJD4B Apa I site DESTROYED |
| A AT A | | pJD4A Apa I site RETAINED |

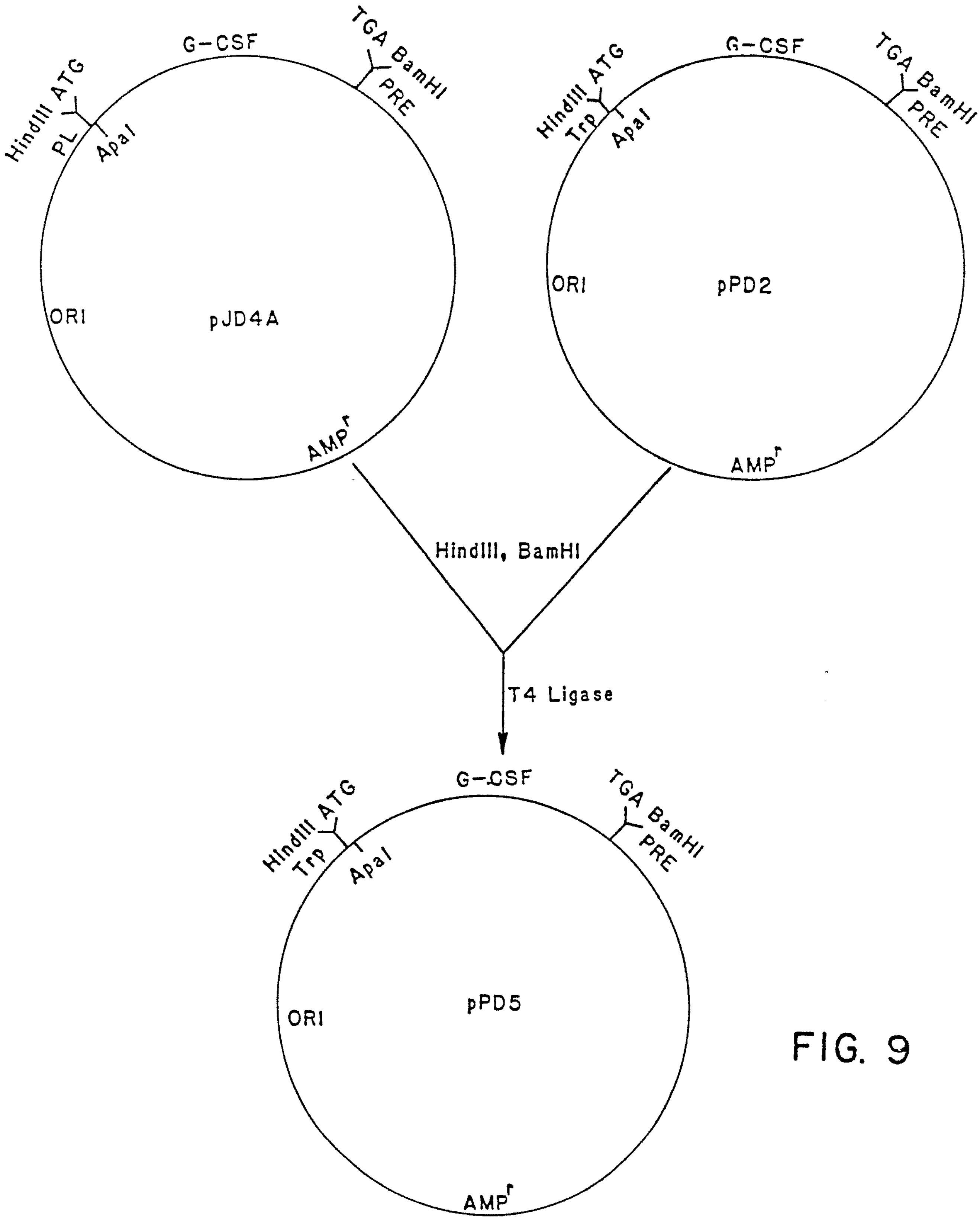
G-CSF
HindIII ATG
PL
ApaI
TGA BamHI
PRE
ORI
pJD4A
AMP$^r$
G-CSF
HindIII ATG
Trp
ApaI
TGA BamHI
PRE
ORI
pPD2
AMP$^r$
HindIII, BamHI
T4 Ligase
G-CSF
HindIII ATG
Trp
ApaI
TGA BamHI
PRE
ORI
pPD5
AMP$^r$

FIG. 9

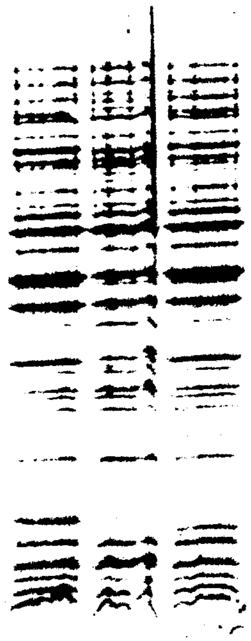

G-CSF
(Met Thr Pro Leu...)
G-CSF
(Pro Leu...)
pJD5 pPD6 pPD6
INDUCED
UNINDUCED
INDUCED


FIG. 15

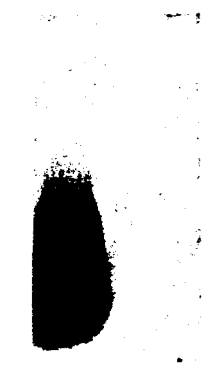

pJD1
pPD2
G-CSF-
-23s
-16s


FIG. 10

0256843

| | 1 | 2 | 3 | 4 | 5 |
|---|---|---|---|---|---|
| | MET | THR | PRO | LEU | GLY |
| G-CSF CODONS | ATG | ACC | CCC | CTG | GGC |
| pJD4B, pPD5 | ATG | ACA | CCA | TTA | GGA |
| pJD4A | ATG | ACA | CCA | TTA | GGC |

FIG. 11

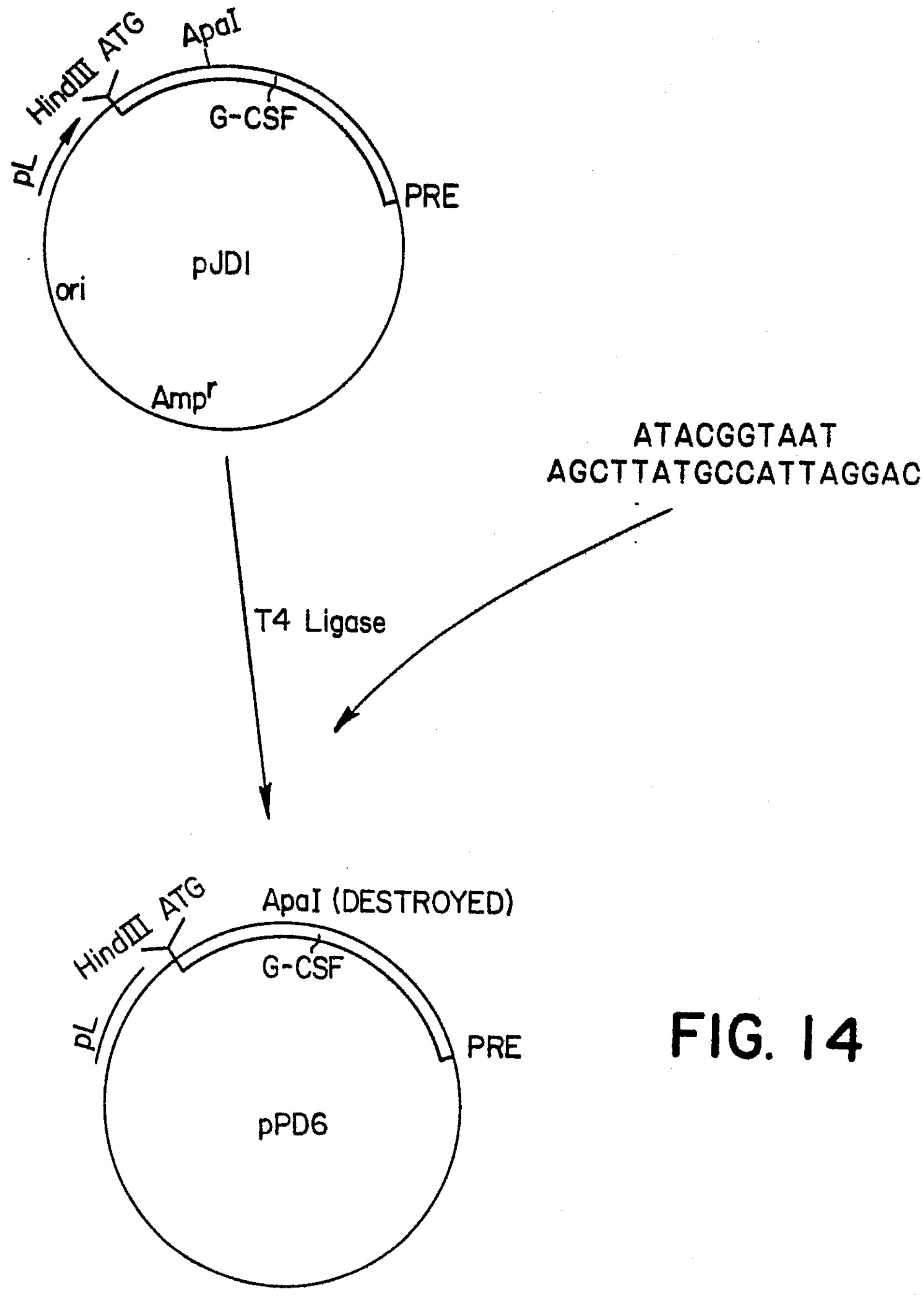


FIG. 14

0256843

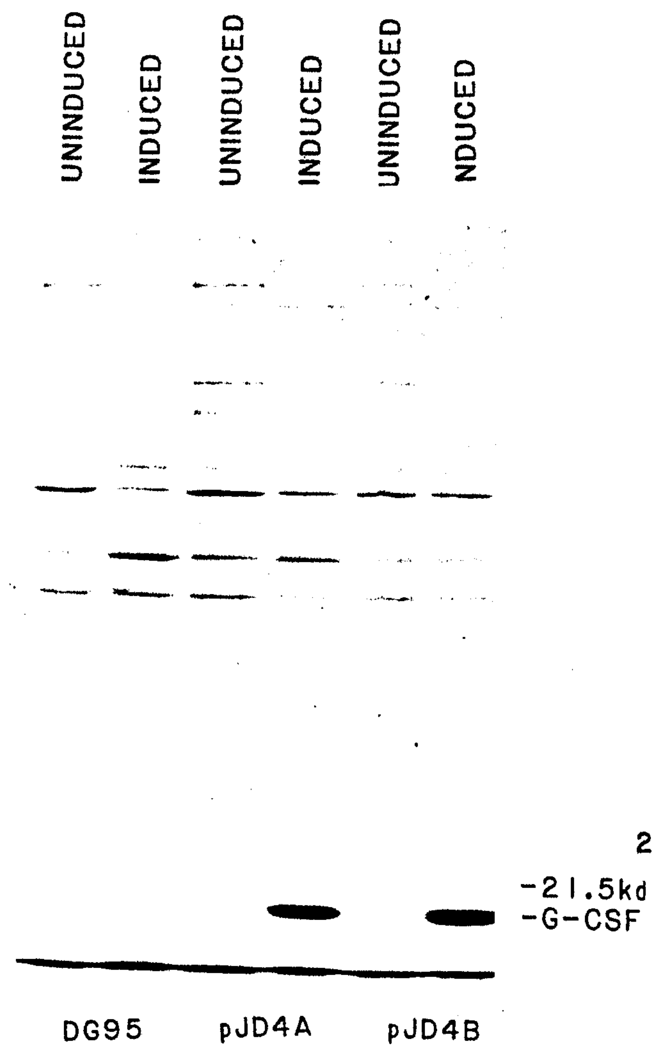


FIG. 12

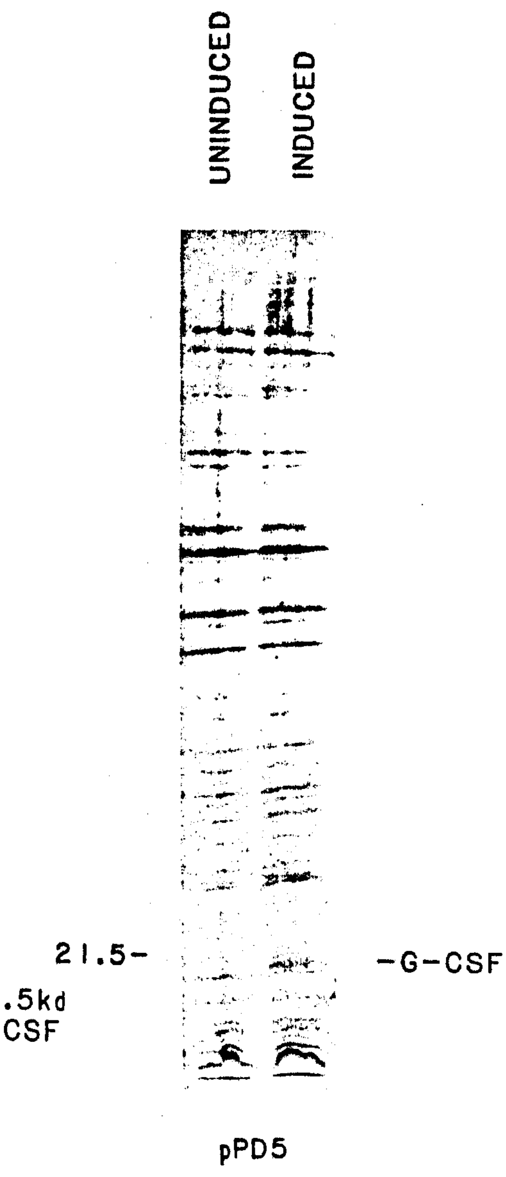


FIG. 13

European Patent Office

# EUROPEAN SEARCH REPORT

0256843
Application Number

EP 87 30 7114

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PROCEEDINGS OF THE NATIONAL ACADEMY OF SCIENCE OF THE USA, vol. 83, October 1986, pages 7633-7637; M. TSUCHIYA et al.: "Isolation and characterization of the cDNA for murine granulocyte colony-stimulating factor" * Whole document * | 1 | C 12 N 15/00<br>C 12 P 21/02 |
| D,Y | SCIENCE, vol. 232, 4th April 1986, pages 61-65: L.M. SOUZA et al.: "Recombinant human granulocyte colony-stimulating factor: effects on normal and leukemic myeloid cells" * Whole document * | 1 | |
| Y | NATURE, vol. 319, no. 6052, January/February 1986, pages 415-418, London, GB; S. NAGATA et al.: "Molecular cloning and expression of cDNA for human granulocyte colony-stimulating factor" * Whole document * | 1 | **TECHNICAL FIELDS SEARCHED (Int. Cl.4)** |
| Y | WO-A-8 600 639 (SANDOZ) * Claims 1-6; pages 18,19 * | 1,6 | C 12 N<br>C 12 P |
| P,X | WO-A-8 701 132 (KIRIN-AMGEN) * Example 8; claims * | 6,9 | |
| P,X | EP-A-0 215 126 (CHUGAI SEIYAKU K.K.) * Claims * | 6,9 | |
| P,X | EP-A-0 220 520 (CHUGAI SEIYAKU K.K.) * Page 6, lines 4-18; claims * | 1,2 | |
| Y | WO-A-8 304 028 (APPLIED MOLECULAR GENETICS) * Claims * | 1 | |

The present search report has been drawn up for all claims

| Place of search | Date of completion of the search | Examiner |
|---|---|---|
| THE HAGUE | 10-11-1987 | DELANGHE L.L.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

Application number: 87307114.6

0256843

DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

IDENTIFICATION OF THE MICRO-ORGANISMS

Accession numbers of the deposits:

ATCC:

67 181
67 183
67 182
39 918
39 946
39 789